(19)

**Europäisches Patentamt**

**European Patent Office**

**Office européen des brevets**

(11) **EP 1 880 656 B1**

(12) **EUROPEAN PATENT SPECIFICATION**

(45) Date of publication and mention
of the grant of the patent:
**22.08.2012 Bulletin 2012/34**

(51) Int Cl.:
*A61B 1/05* (2006.01)     *A61B 1/12* (2006.01)
*G02B 23/24* (2006.01)

(21) Application number: **06728912.4**

(86) International application number:
**PCT/JP2006/304744**

(22) Date of filing: **10.03.2006**

(87) International publication number:
**WO 2006/120797 (16.11.2006 Gazette 2006/46)**

(54) **DISTAL END PORTION OF ENDOSCOPE**

DISTALER ENDBEREICH EINES ENDOSKOPES

PARTIE D'EXTREMITE DISTALE D'UN ENDOSCOPE

(84) Designated Contracting States:
**DE FR GB**

(30) Priority: **11.05.2005 JP 2005138652**

(43) Date of publication of application:
**23.01.2008 Bulletin 2008/04**

(73) Proprietor: **OLYMPUS MEDICAL SYSTEMS CORP.**
**Tokyo 151-0072 (JP)**

(72) Inventor: **ICHIMURA, Hironobu**
**c/o Intellectual Property Support Department**
**Hachioji-shi**
**Tokyo 192-8512 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**WO-A1-96/25874     JP-A- 2005 000 640**
**JP-U- 2 053 701     JP-U- 3 056 402**
**US-A- 5 730 701     US-A- 5 782 751**
**US-A- 5 860 913     US-A1- 2004 158 129**

## Description

Technical Field

The present invention relates to a distal end portion of an endoscope provided with an observation optical system of an object-contacting type where a distal end portion of an objective optical system is brought in contact with an object, where the object is observed.

Background Art

Jpn. Pat. Appln. KOKAI Publication No. 2005-640 (Patent document 1) discloses an endoscope provided with an observation optical system of an object-contacting type and an ordinary observation optical system. In the observation optical system of the object-contacting type, a distal end portion of an objective optical system is brought in contact with an object, where the object is observed. In the ordinary observation optical system, the objective optical system is put in a non-contacting state with an object, where the object is observed.

Jpn. Pat. Appln. KOKAI Publication No. 2002-325722 (Patent document 2) discloses an endoscope where a projecting portion caused to project forward is provided on a distal end face of an insertion portion of the endoscope. An observation window for the observation optical system, an illumination window for an illumination optical system, an air-supplying/water-supplying nozzle, and a dirty mucosa cleaning nozzle are disposed on an end face of the projecting portion. The dirty mucosa cleaning nozzle jets water for cleaning a dirty mucosa face. Here, arrangement is made such that a distal end portion of the air-supplying/water-supplying nozzle is opened toward the observation window of the observation optical system. Air or water jetted from the distal end opening portion of the air-supplying/water-supplying nozzle is sprayed on the observation window for the observation optical system to remove dirt on the observation window. Further, arrangement is made on an end face of the projecting portion of the insertion portion on a root side thereof such that a distal end opening portion of a procedure tool insertion channel is opened forward.

Documents US 5,782,751, US 5,860,913 and US 5,730,701 concern side-view type endoscopes each having a distal end portion with a structure different from that of the distal end portion according to the present invention.

Document US 2004/0158129 discloses an endoscope with an inserting tube, first and second optical systems integrally secured to a tip end of the inserting tube. Said optical means observe the in vivo tissues at different magnifications. A front end portion of the second optical system is protruded by a predetermined amount with respect to the first optical system so that the position of the second optical system is localizable by the field of view of the second optical system. .

Disclosure of Invention

In Patent document 2, the air-supplying/water-supplying nozzle is disposed on an end face of the projecting portion caused to project forward at a distal end of the insertion portion of the endoscope. A distal end portion of the supplying/water-supplying nozzle is caused to project forward beyond a distal end portion position of the observation optical system of the object-contacting type. In this case, when the observation optical system of the object-contacting type is brought in contact with an object to observe the object, such a possibility arises that the opening portion of the air-supplying/water-supplying nozzle is caught by the object. As a result, there is a possibility that it becomes difficult to conduct work for bringing the observation optical system of the object-contacting type in contact with the object or it becomes difficult to make determination about pathology because a body tissue surface on an observation site gets scratched.

The present invention has been made in view of the above circumstances, and an object thereof is to provide a distal end portion of an endoscope where a possibility that an opening portion of an air-supplying/water-supplying nozzle is caught by an object can be reduced and observation or diagnosing work utilizing an observation optical system of an object-contacting type can be performed easily.

A distal end portion of an endoscope according to the present invention comprises the features as defined in claim 1. insertion portion for delivering fluid to the observation portion.

With the configuration of the above one aspect, by arranging the nozzle portion for delivering fluid to the observation portion provided on the distal end portion of the insertion portion on the non-projecting face of the insertion portion, the height of the nozzle is lowered. Thereby, when the projecting face of the insertion portion is brought in contact with a body tissue (a body to be examined) to conduct observation, the nozzle portion is made less likely to be caught by a living body.

A distal end portion of an endoscope not part of the invention, has been also described, comprising : an insertion portion to be inserted into a body to be examined; a projecting face which is provided at a distal end portion of the insertion portion in a projecting manner and on which a first observation portion for observing the body to be examined is disposed; and a nozzle portion which is provided on a non-projecting face of the insertion portion for delivering fluid to a second observation portion disposed on the non-projecting face.

A distal end portion of an endoscope not part of the invention, has been also described, comprising: an insertion portion to be inserted into a body to be examined; a first distal end face which is provided at a distal end portion of the insertion portion and on which a first observation portion for observing the body to be examined is disposed; a second distal end face which is provided on a distal end side to the first distal end face in a projecting manner and on which a second observation portion for observing the body to be examined is disposed; and a nozzle portion for delivering fluid to the first observation portion disposed on the first distal end face.

A distal end portion of an endoscope not part of the invention, has been also described, comprising. an insertion portion to be inserted into a body to be examined; a first distal end face which is provided at a distal end portion of the insertion portion and on which a first observation portion for observing the body to be examined is disposed; a second distal end face which is provided on a proximal end side to the first distal end face and on which a second observation portion for observing the body to be examined is disposed; and an opening portion for delivering a fluid toward a distal end side provided on the second distal end face.

A distal end portion of an endoscope not part of the invention, has been also described, comprising: an insertion portion to be inserted into a body to be examined; a first distal end face which is provided at a distal end portion of the insertion portion and on which a first observation portion for observing the body to be examined is disposed; a second distal end face which is provided on a distal end side to the first distal end face in a projecting manner and on which a second observation portion for observing the body to be examined is disposed; a third distal end face which is provided on a proximal end side to the first distal end face; and a nozzle portion provided on the third distal end face for delivering a fluid to the first observation portion.

According to the present invention, since the height of the air-supplying/water-supplying nozzle becomes low, such a distal end portion of an endoscope can be provided that, when observation is made in a contacting with a body tissue (a body to be examined), a possibility that the air-supplying/water-supplying nozzle is caught by an object can be reduced and observation or diagnosing work utilizing an observation optical system of an object-contacting type can be performed easily.

Brief Description of Drawings

FIG. 1 is a schematic configuration diagram of the entire system of an endoscope according to a first embodiment of the present invention;
FIG. 2 is a front view of a distal end portion of the endoscope according to the first embodiment;
FIG. 3 is a vertical sectional view showing an optical system incorporated in the distal end portion of the endoscope according to the first embodiment;
FIG. 4A is a vertical sectional view showing an observation optical system for ordinary observation assembled in the distal end portion of the endoscope according to the first embodiment;
FIG. 4B is a vertical sectional view showing a coupling portion between a bending portion and a flexible tube portion of the endoscope;
FIG. 5A is a sectional view taken along line VA - VA in FIG. 4A;
FIG. 5B is a sectional view taken along line VB - VB in FIG. 4A;
FIG. 6 is a vertical sectional view of a main section showing a configuration of an air-supplying/water-supplying nozzle of the endoscope according to the first embodiment;
FIG. 7 is a vertical sectional view of a main section showing a configuration of the observation optical system for ordinary observation of the endoscope according to the first embodiment;
FIG. 8A is a vertical sectional view of a whole unit showing the observation optical system for ordinary observation of the endoscope according to the first embodiment;
FIG. 8B is a vertical sectional view of a main section showing a state that a zoom optical system has been moved up to a stopper position in a wide angle direction;
FIG. 8C is a vertical sectional view of a main section showing a state that a brightness aperture between lenses has been attached;
FIG. 9A is a vertical sectional view of a main section showing a state that movement has been conducted up to a stopper position in an enlargement direction at a zoom action time of the observation optical system for ordinary observation of the endoscope according to the first embodiment;
FIG. 9B is a vertical sectional view of a main section showing a state that movement has been conducted up to a stopper position in a wide angle direction;
FIG. 10 is a vertical sectional view of a main section showing a jig for assembling the observation optical system for ordinary observation of the endoscope according to the first embodiment;
FIG. 11A is a vertical sectional view showing a whole unit of an observation optical system of an object-contacting type of the endoscope according to the first embodiment;
FIG. 11B is a vertical sectional view of an optical unit of the observation optical system of an object-contacting type;
FIG. 12 is a vertical sectional view of a main section of the distal end portion of the endoscope according to the first embodiment;
FIG. 13 is a vertical sectional view showing a configuration of forward water-supplying pipe conduit incorporated in the distal end portion of the endoscope according to the first embodiment;
FIG. 14 is an explanatory diagram for explaining a flow state of water flow from an air-supplying/water-supplying nozzle at the distal end portion of the endoscope according to the first embodiment;
FIG. 15 is a vertical sectional view of a main section showing an observation state obtained by the observation optical system of an object-contacting type of the endoscope according to the first embodiment;

FIG. 16A is an explanatory diagram for explaining an observation state obtained by the observation optical system of an object-contacting type of the endoscope according to the first embodiment;

FIG. 16B is an explanatory diagram for explaining a relationship between a height of a projecting step portion and an incident angle of incident light into a first lens for a second imaging unit for ordinary observation;

FIG. 17 is an explanatory diagram for explaining an action for cleaning a clogged portion in a forward water-supplying pipe conduit of the endoscope according to the first embodiment;

FIG. 18 is a characteristic diagram for explaining difference in observation state due to the difference in distance between the center position of an objective lens and the center position of an illumination window for illumination light at a time of observation performed by the observation optical system of an object-contacting type of the endoscope according to the first embodiment;

FIG. 19 is a front view of a distal end portion of an endoscope according to a second embodiment of the present invention;

FIG. 20 is a vertical sectional view showing an optical system incorporated in the endoscope according to the second embodiment;

FIG. 21 is a vertical sectional view of a main section showing a configuration of an observation optical system for ordinary observation of the endoscope according to the second embodiment;

FIG. 22 is a vertical sectional view of a main section showing a configuration of an air-supplying/water-supplying nozzle of the endoscope according to the second embodiment;

FIG. 23 is a front view of a distal end portion of an endoscope according to a third embodiment of the present invention;

FIG. 24 is a front view of a distal end portion of an endoscope according to a fourth embodiment of the present invention;

FIG. 25 is a front view of a distal end portion of an endoscope according to a fifth embodiment of the present invention;

FIG. 26 is a front view of a distal end portion of an endoscope according to a sixth embodiment of the present invention;

FIG. 27 is a front view of a distal end portion of an endoscope according to a seventh embodiment of the present invention; and

FIG. 28 is a front view of a distal end portion of an endoscope according to example useful for understanding the present invention.

Best Mode for Carrying Out the Invention

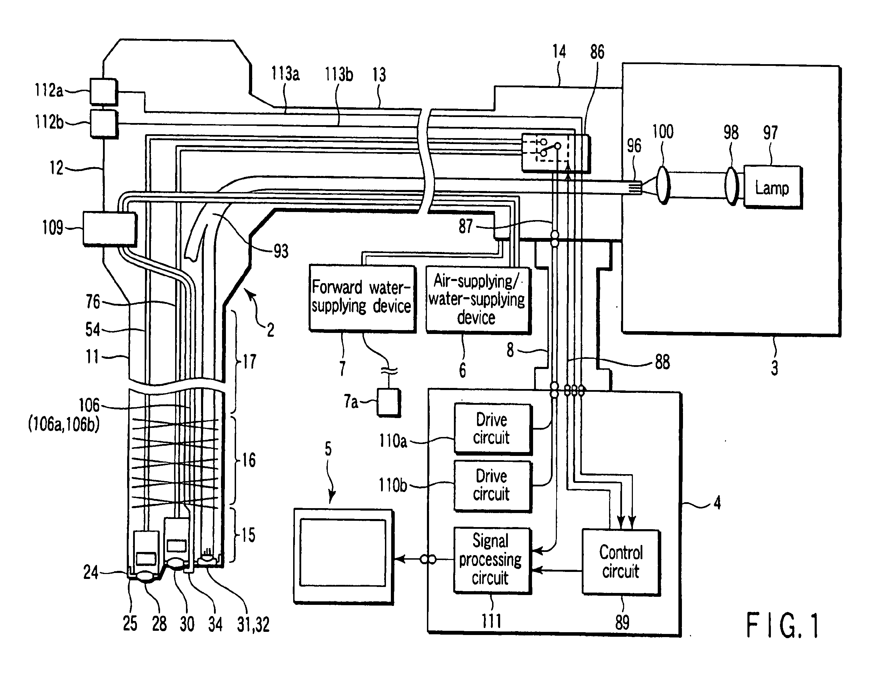

A first embodiment of the present invention will be explained below with reference to FIG. 1 to FIG. 18. FIG. 1 shows a schematic configuration of a whole endoscope system 1 of the present embodiment. As shown in FIG. 1, the endoscope system 1 according to the embodiment is provided with an endoscope 2, a light source apparatus 3, a processor 4, a monitor 5, an air-supplying/water-supplying apparatus 6, and a forward water-supplying apparatus 7. The light source apparatus 3 is illuminating means for supplying illumination light to the endoscope 2. The processor 4 is a signal processing device that performs signal processing for the endoscope 2. The monitor 5 is connected to the processor 4. The air-supplying/water-supplying apparatus 6 performs air supply and water supply. The forward water-supplying apparatus 7 performs forward water supply.

The endoscope 2 includes a slender insertion portion 11 to be inserted into a body cavity, an operation portion 12 coupled to a proximal end of the insertion portion 11, and a universal cable 13 extending from a side portion of the operation portion 12. A connector 14 is provided at an end portion of the universal cable 13. The connector 14 is attachably and detachably connected to the light source apparatus 3. Further, the connector 14 is connected with one end portion of a scope cable 8. The other end portion of the scope cable 8 is connected to the processor 4.

Further, the insertion portion 11 of the endoscope 2 includes a hard distal end portion 15 formed at a distal end thereof, a bending portion 16 formed at a proximal end of the distal end portion 15, and a flexible tube portion 17 with flexibility formed so as to extend from a proximal end of the bending portion 16 to the operation portion 12.

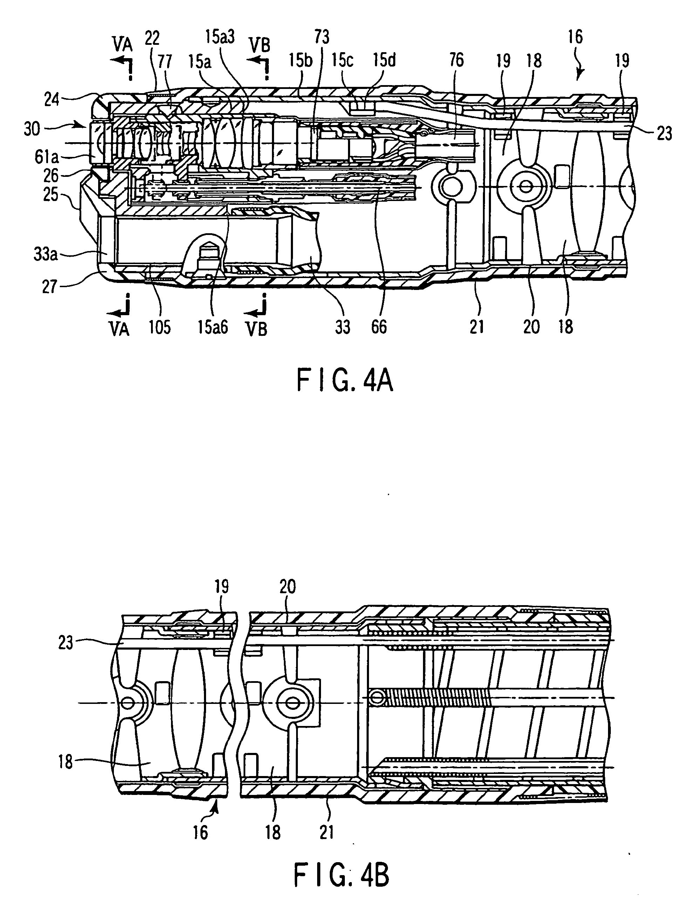

As shown in FIGS. 4A and 4B, a plurality of ring-like bending pieces 18 are continuously provided rotatably along an axial direction of the insertion portion 11. Each bending piece 18 is fixedly provided with four pipe-like wire receivers 19 on an inner peripheral face thereof by means of welding or the like. The four wire receivers 19 are fixed on an inner peripheral face of one bending piece 18 at positions respectively shifted around an insertion axis at intervals of about 90°.

A bending blade 20 formed by knitting thin wires in a cylindrical shape is capped on the plurality of bending pieces 18 so as to cover outer peripheries thereof. An outer skin 21 is capped on the bending blade 20 so as to maintain water-tightness.

The outer skin 21 integrally covers the insertion portion 11 comprising the distal end portion 15, the bending portion 16, and the flexible tube portion 17 over the whole length thereof. After a distal end outer peripheral portion of the outer skin 21 is wound on the distal end portion 15 in a spool manner, it is fixed by a spool adhesion portion 22.

Further, four bending operation wires 23 for operating the bending portion 16 in a bending manner are inserted in the insertion portion 11. Distal end portions of these four bending operation wires 23 are held and fixed by four fixing portions 15d of the fixing ring 15c provided in the distal end portion 15 while being shifted around the insertion axis at intervals of about 90°. Further, each of four bending operation wires 23 is inserted into each wire receiver 19 on the inner peripheral face of the bending piece 18. Each of the four bending operation wires 23 extends from the bending portion 16 toward the operation portion 12 on the proximal end side through the inside of the flexible pipe portion 17. The fixing ring 15c is inserted and fitted on the inner peripheral side of a reinforcing ring 15b in the distal end portion 15 described later.

Incidentally, the distal end portion 15 and the each bending piece 18 are coupled to each other such that each bending operation wire 23 that is inserted into each wire receiver 19 of each bending piece 18 in a state that the insertion axis of the bending portion 16 is approximately straight becomes approximately linear.

These bending operation wires 23 are coupled to a bending operation mechanism (not shown) whose proximal end portion is provided inside the operation portion (see FIG. 1). Two bending operation knobs for four-directions bending operation (not shown) for driving the bending operation mechanism is disposed on the operation portion 12.

Four bending operation wires 23 are alternately pulled or loosened according to the operation of the bending operation knobs so that the bending portion 16 is operated in four directions in a bending manner. These four directions are four directions of upward, downward, leftward, and rightward directions of an endoscope image displayed on the monitor 5 described later.

Two bending operation wires 23 that constitute first bending operation means for operating the bending portion 16 in upward and downward directions and two bending operation wires 23 that constitute second bending operation means for operating the bending portion 16 in leftward and rightward directions are paired, respectively. That is, two bending operation wires 23 inserted and held in two wire receivers 19 in directions corresponding to the upward and downward directions in the bending pieces 18 inside the bending portion 16 constitute the first bending operation means for upward and downward direction operations. Two bending operation wires 23 inserted and held in two wire receivers 19 in directions corresponding to the leftward and rightward directions in the bending pieces 18 inside the bending portion 16 constitute the second bending operation means for leftward and rightward direction operations.

Incidentally, the upward and downward directions as a first direction explained later will be explained as upward and downward directions of an endoscope image displayed on the monitor 5 or upward and downward directions in which the bending portion 16 is operated in a bending manner. Ordinarily, the monitor 5 is set such that its upward and downward directions approximately coincide with vertically upward and downward directions. Further, leftward and rightward directions that are a second direction approximately orthogonal to the above upward and downward directions are equal to the leftward and rightward directions of an endoscope image displayed on the monitor 5 and the leftward and rightward directions in which the bending portion 16 is operated in a bending manner.

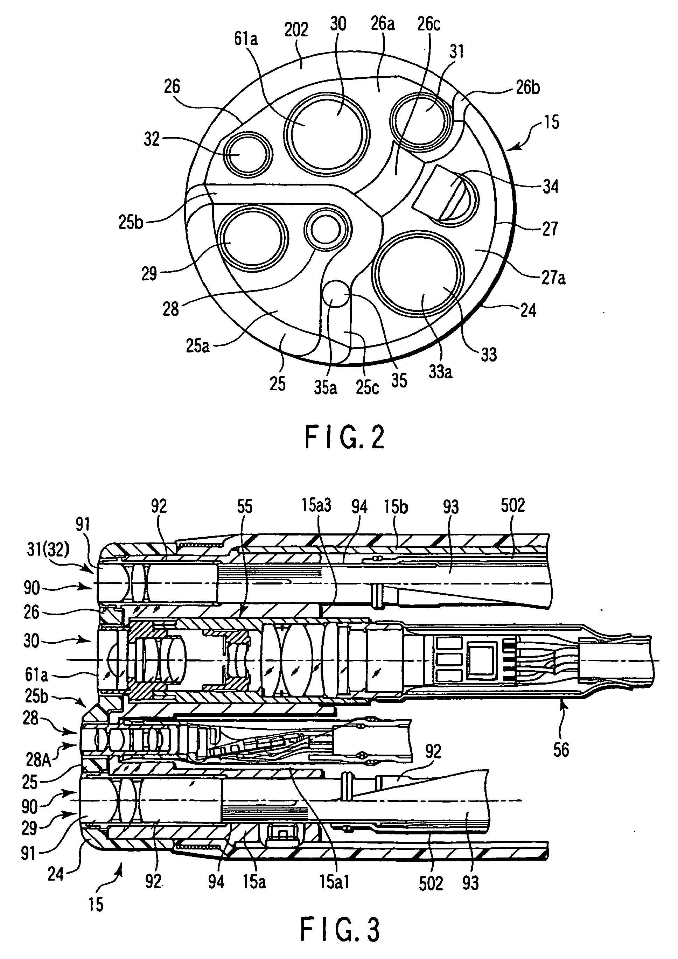

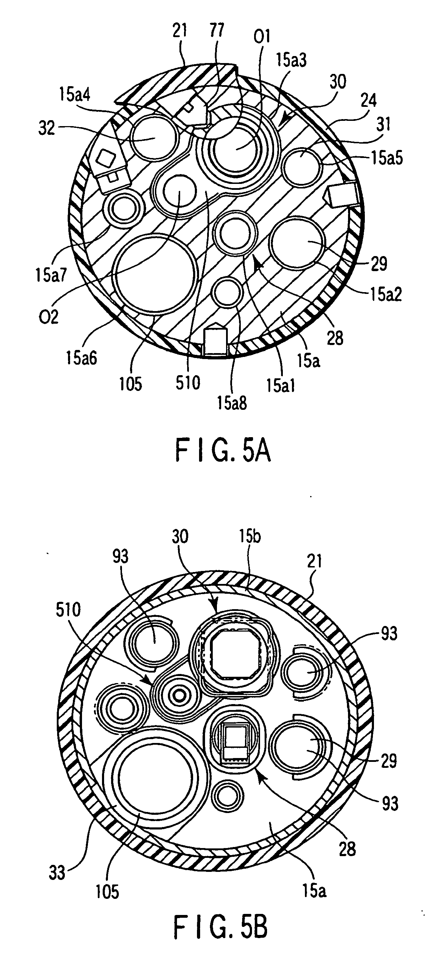

FIG. 3 and FIG. 4A shows an internal configuration of the distal end portion of the insertion portion 11 of the endoscope 2 according to the present embodiment. As shown in FIG. 3, a column-shaped member (a distal end hard member) 15a made from hard metal and an annular reinforcing ring 15b fitted on a proximal end outer peripheral portion of the column-shaped member 15a are disposed in the distal end portion 15 of the insertion portion 11. As shown in FIG. 5, a plurality of (eight (first to eighth) in the present embodiment) hole portions 15a1 to 15a8 parallel to the axial direction of the insertion portion 11 are formed in the column-shaped member 15a. A proximal end portion of the reinforcing ring 15b is coupled to the most-advanced bending piece 18.

Further, a distal end cover 24 is attached so as to be fitted on a distal end face of the column-shaped member 15a and the distal end outer peripheral portion of the column-shaped member 15a. As shown in FIG. 2, step portions 25, 26, and 27 of three steps comprising a projecting step portion 25 projecting forward, a middle step portion 26 lower than the projecting step portion 25 by one step, and a low step portion 27 lower than the middle step portion 2b by one step are formed on the distal end cover 24 disposed at the distal end portion 15 of the insertion portion 11. Here, an end face of the projecting step portion (projecting portion) 25 is formed by a flat face 25a orthogonal to the axial direction of the insertion portion 11. A projecting face is formed by the flat face 25a of the projecting step portion 25.

In the present embodiment, the flat face 25a of the projecting step portion 25 is formed to have an area of about 1/4 of a cylindrical shape of the whole circular front face of the distal end cover 24. That is, the flat face 25a is formed on a lower half portion of the whole circular front face of the distal end cover 24 on a left side portion regarding a centerline connecting an upper side and a lower side in FIG. 2.

A first lens 41a, which is an observation lens for a first imaging unit (first observing unit) 28 of an object-contacting type, described later, and a first illumination window 29 are disposed on the flat face 25a of the projecting step portion 25. The first imaging unit 28 is disposed at an approximately central position of the distal end portion 15. The first illumination window 29 is disposed at a position near the first imaging unit 28.

The middle step portion 26 has a flat face 26a approximately parallel to the flat face 25a of the projecting step portion 25. A first lens 61a, which is an observation lens for a second imaging unit (second observing unit) 30 for ordinary observation described later, and two (second and third) illumination windows 31 and 32 are disposed on the flat face 26a of the middle step portion 26. Here, the second and third illumination windows 31 and 32 are disposed on both sides of the second imaging unit 30. Further, an inclined face 25b with an inclination angle of, for example, about 45° is formed on a wall portion between the middle step portion 26 and the projecting step portion 25.

Incidentally, a step between the flat face 25a of the projecting step portion 25 and the flat face 26a of the middle step portion 26 is set to a height which can prevent the projecting step portion 25 from entering the field of view of the second imaging unit 30, for example, a height of about 0.7 mm.

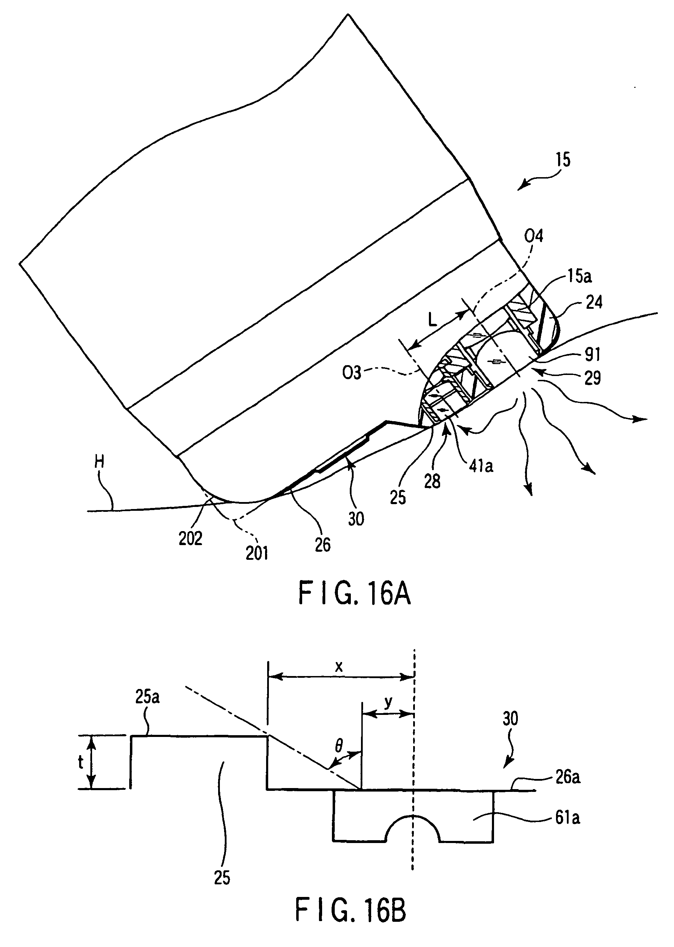

FIG. 16B is an explanatory diagram for explaining a relationship between the height of the projecting step portion 25 and an incident angle θ of incident light into the first lens 61a of the second imaging unit 30 for ordinary observation. Here, parameters are as follows: The parameter "x" represents a distance from a lens face center of the first lens 61a to the projecting step portion 25, "θ" represents an incident angle of incident light into the first lens 61a, "y" represents a beam height of a lens first face, and "t" represents a height of the projecting step portion 25.

A relational expression showing a relationship between the height of the projecting step portion 25 and the incident angle θ of the incident light of the first lens 61a for the second imaging unit 30 for ordinary observation is shown by the following expression (1).

$$\tan \theta = (x - y)/t \quad ... \quad (1)$$

Therefore, since the projecting step portion 25 does not enter the field of view of the first lens 61a for the second imaging unit 30, setting to a size smaller than a value of t of the following expression (2) may be conducted.

$$t = (x - y)/\tan \theta \quad ... \quad (2)$$

For example, when setting of θ = about 70°, x = about 3.5 mm, and y = about 1 mm is performed as "parameters", t = 0.91 mm is obtained as "calculation result". Thereby, it is understood that, when setting to a size smaller than a value of t = 0.91 mm is conducted in a case of the above "parameters", the projecting step portion 25 does not enter the field of view of the first lens 61a for the second imaging unit 30.

Incidentally, three illumination windows arranged at the distal end portion 15 of the insertion portion 11, namely, the first illumination window 29 disposed on the flat face 25a of the projecting step portion 25, and the second and third illumination windows 31 and 32 disposed on the flat face 26a of the middle step portion 26 are set to satisfy the following relationship. In the present embodiment, setting is conducted such that the first illumination window 29 has the largest area, the second illumination window 31 has the second largest area, and the third illumination window 32 has the smallest area. Thereby, outgoing light quantities from three illumination windows are set such that the first illumination window 29 has the most outgoing light quantity, the second illumination window 31 has the second most outgoing light quantity, and the third illumination window 32 has the least outgoing light quantity.

In the present embodiment, the first lens 61a serving as the observation lens disposed at the distal end of the second imaging unit 30 for ordinary observation is set to be larger in lens diameter (external diameter) than the first lens 41a serving as the observation lens disposed at the distal end of the first imaging unit 28.

The low step portion 27 includes a flat face 27a approximately parallel to the flat face 25a of the projecting step portion 25. A distal end opening portion 33a of a procedure tool insertion channel (also called "forceps channel") 33 disposed inside the insertion portion 11 and an air-supplying/water-supplying nozzle 34 described later are disposed on the flat face 27a of the lower step portion 27.

Further, an inclined face 26b with an inclination angle of, for example, about 45° and a fluid guide face 26c with an inclination angle smaller than that of the inclined face 26b are formed on a wall portion provided between the lower step portion 27 and the middle step portion 26. The fluid guide face 26c is disposed between the air-supplying/water-supplying nozzle 34 on the lower step portion 27 and the second imaging unit 20 on the middle step portion 26. The fluid guide face 26c is formed by a gentle inclined face with an inclination angle of, for example, about 18°.

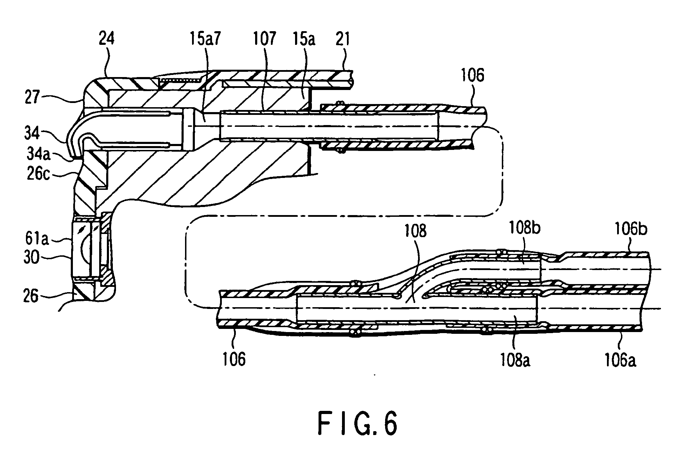

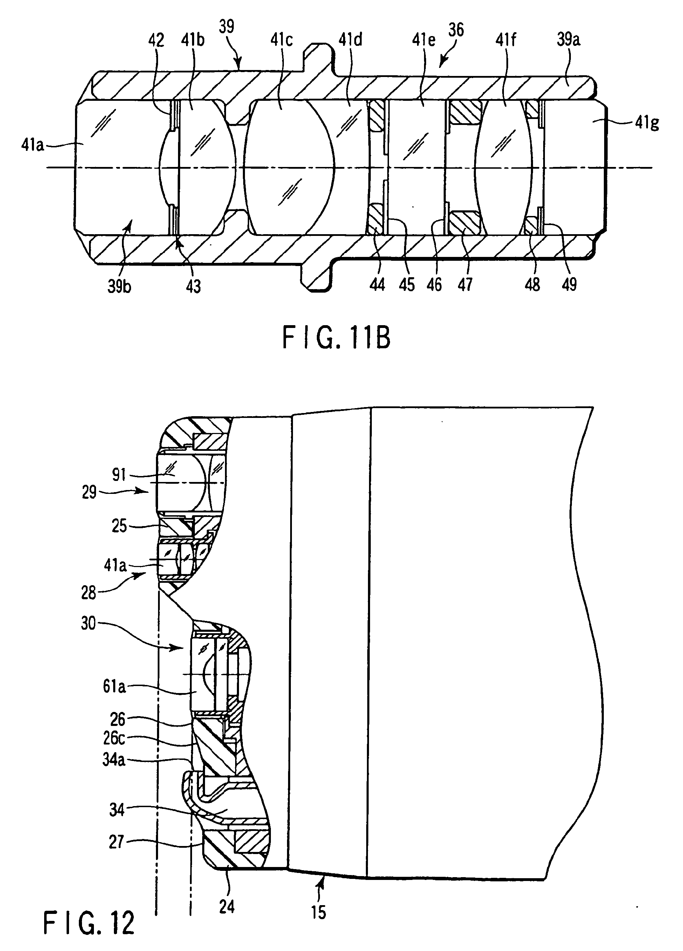

As shown in FIG. 6, the air-supplying/water-supplying nozzle 34 is a pipe-like member bend in an approximate L shape. Arrangement is made such that a distal end portion of the air-supplying/water-supplying nozzle 34 is directed to the first lens 61a side, which is the observation lens for the second imaging unit 30. Further, an opposing arrangement is made such that a jetting port 34a at a distal end opening portion of the air-supplying/water-supplying nozzle 34 is directed to the fluid guide face 26c. Here, as shown in FIG. 12, a distal end face of the jetting port 34a of the distal end opening portion of the air-supplying/water-supplying nozzle 34 and the first lens 61a, which is the observation lens for the second imaging unit 30, are arranged on approximately the same face. Thereby, the draining property at a time of cleaning can be improved.

Incidentally, the air-supplying/water-supplying nozzle 34 is connected to an air-supplying/water-supplying pipe conduit 106 through joining of a distal end of the former to the latter, and a proximal end side of the air-supplying/water-supplying pipe conduit 106 is branched to an air-supplying pipe conduit 106a and a water-supplying pipe conduit 106b, as described later.

A non-projecting face is formed by a portion of the projecting step portion 25 except for a projecting face which is the flat face 25a of the projecting step portion 25. The non-projecting face is formed by, for example, the flat face 26a on the middle step portion 26, the flat face 27a on the low step portion 27, the inclined face 25b on the wall portion between the middle step portion 26 and the projecting step portion 25, the inclined face 26b and the fluid guide face 26c on the wall portion between the low step portion 27 and the middle portion 26, the inclined face 25c on the wall portion between the low step portion 27 and the projecting step portion 25, and the like. The inclined face 25c is formed to have an inclination angle of, for example, about 45°.

Here, as shown in FIG. 12, the projecting face, which is the flat face 25a of the projecting step portion 25, is disposed on a distal end side of the air-supplying/ water-supplying nozzle 34 beyond the distal end portion thereof. Thereby, when the flat face 25a of the projecting step portion 25 is caused to abut on a body to be examined, the distal end portion of the air-supplying/water-supplying nozzle 34 is prevented from being caught by the body to be examined.

Further, an opening portion 35a for forward water supply is disposed on a non-projecting face, or on the inclined face 25c between the low step portion 27 and the projecting step portion 25 in the present embodiment at the distal end portion 15 of the insertion portion 11. As shown in FIG. 2, the opening portion 35a for this forward water supply is disposed on near a vertical central axis of the second imaging unit 30 for ordinary observation. The opening portion 35a is caused to communicate with a pipe conduit (forward water-supplying channel) 35 for forward water supply inserted into the insertion portion. Incidentally, an inner diameter of the pipe conduit 35 for forward water supply is set to about 1 mm.

The eight (the first to eighth) hole portions 15a1 to 15a8 of the column-shaped member 15a in the distal end portion 15 are provided at positions corresponding to the first imaging unit 28, the first illumination window 29, the second imaging unit 30, the second illumination window 31, the third illumination window 32, the distal end opening portion 33a of the procedure tool insertion channel 33, the air-supplying/water-supplying nozzle 34, and the opening portion 35a for forward water-supply of the distal end cover 24, respectively. Constituent elements of the first imaging unit 28, constituent elements of the first illumination window 29, constituent elements of the second imaging unit 30, constituent elements of the second illumination window 31, constituent elements of the third illumination window 32, constituent elements of the pipe conduit of the procedure tool insertion channel 33, constituent elements of the pipe conduit for the air-supplying/water-supplying nozzle 34, and constituent elements of the pipe conduit communicating with the opening portion 35a for forward water supply are assembled to the first hole portion 15a1, the second hole portion 15a2, the third hole portion 15a3, the fourth hole portion 15a4, the fifth hole portion 15a5, the sixth hole portion 15a6, the seventh hole portion 15a7, and the eighth hole portion 15a8, respectively, as described later.

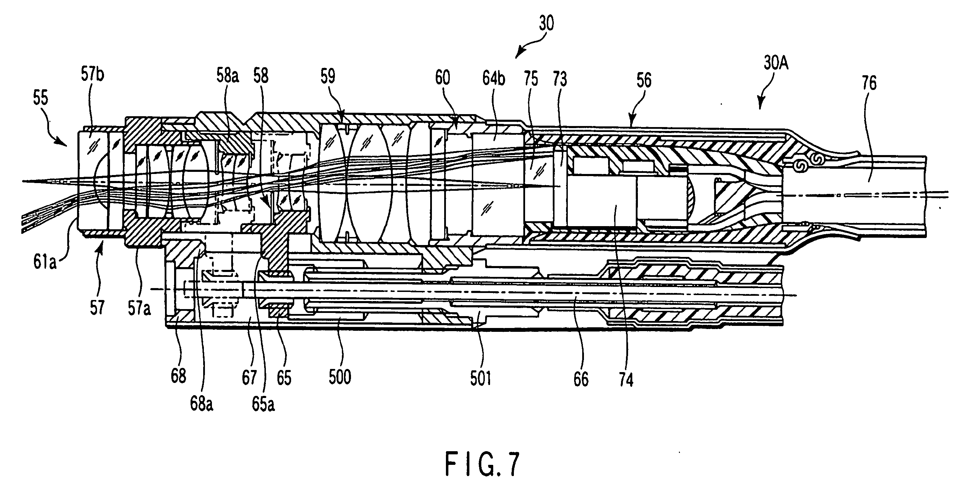

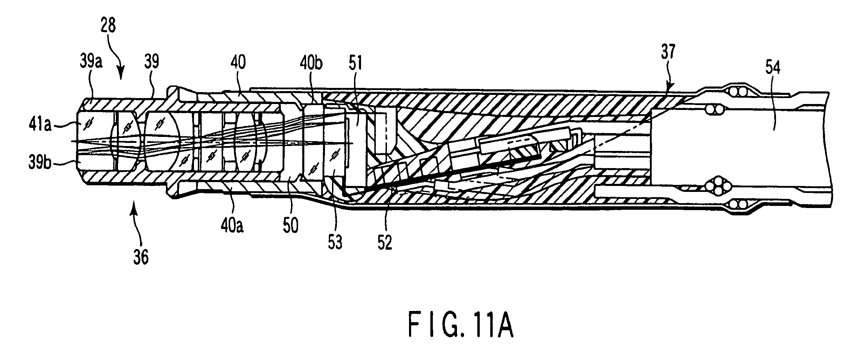

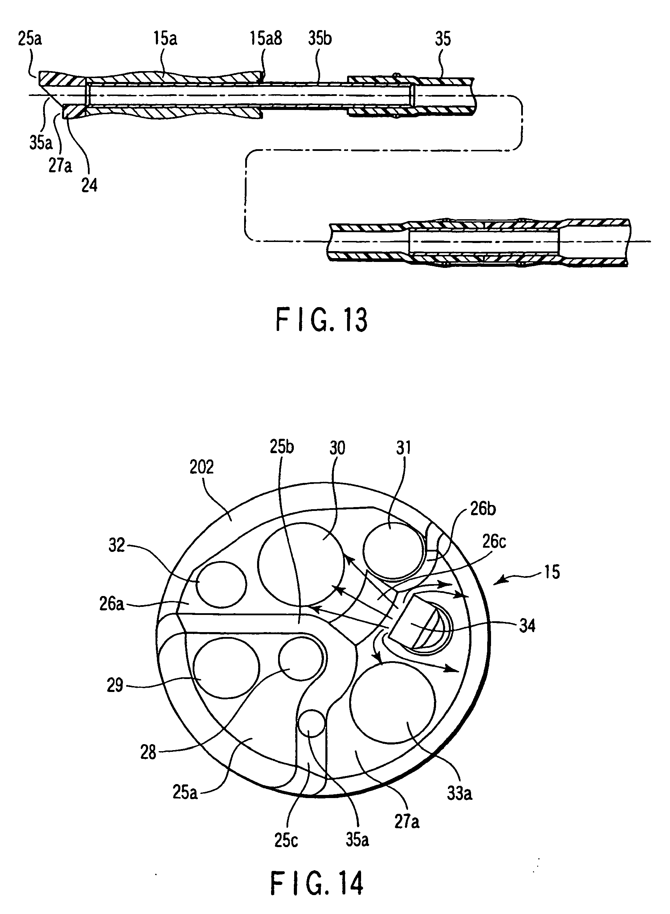

FIG. 11A shows a configuration of the first imaging unit 28 of an object-contacting type, FIG. 7 shows a configuration of the second imaging unit 30 for ordinary observation, FIG. 6 shows a configuration of the air-supplying/water-supplying nozzle 34, and FIG. 13 shows a configuration of the opening portion 35a for forward water supply, respectively.

As shown in FIG. 11A, the first imaging unit 28 includes a first lens unit 36 with a super high magnification and a first electric part unit 37. Incidentally, the super high magnification of the first lens unit 36 is a magnification with a histological observation level including a cell or a structure of duct of the gland (a level nearly equal to the magnifying power of a middle-level ordinary optical microscope, for example, about 200 to 1000 times).

The first lens unit 36 further includes two (first and second) unit configuration bodies 39 and 40. The first lens unit configuration body 39 includes a first lens frame 39a and a first lens group 39b. As shown in FIG. 11B, the first lens group 39b includes seven (first to seventh) objective lenses 41a to 41g. Here, the first lens 41a, which is an observation lens, is disposed at a distal end portion of the first lens frame 39a. For example, adhesion and fixation to the first lens frame 39a are performed in a state that the distal end portion of the first lens 41a projects forward beyond a distal end portion of the first lens frame 39a.

An optical aperture 42 and an adjusting aperture 43 for adjusting a distance between lens faces are interposed between the first lens 41a and the second lens 41b positioned behind the first lens 41a. Further, the third lens 41c to the seventh lens 41g are sequentially disposed behind the second lens 41b. Here, a spacer ring 44 and an optical aperture 45 are interposed between the fourth lens 41d and the fifth lens 41e. Further, an optical aperture 46 and a spacer ring 47 are interposed between the fifth lens 41e and the sixth lens 41f. A spacer ring 48 and an adjusting aperture 49 are interposed between the sixth lens 41f and the seventh lens 41g.

The second unit configuration body 40 includes a second lens frame 40a and a second lens 40b. The second lens 40b is disposed in the second lens frame 40a behind an accommodating space 50 accommodating the first unit configuration body 39 therein.

The first electric part unit 37 is continuously provided at a rear end portion of the first lens unit 36. Here, the first electric part unit 37 includes a first imaging device 51 such as a CCD (charge coupled device) or a CMOS (complementary metal-oxide semiconductor), and a first circuit board 52. Further, a cover lens 53 is provided on a light receiving face side of a front face of the first imaging device 51.

The cover lens 53 of the first electric part unit 37 is fixed in a state that it is provided in parallel to an objective lens at a rear end portion of the first lens unit 36, namely, the second lens 40b of the second unit configuration body 40. Thereby, an observation optical unit 28A with a super high magnification obtained by integrating the first lens unit 36 and the first electric part unit 37 is formed.

The first circuit board 52 includes electric parts and a wiring pattern, and it is connected with distal end portions of a plurality of signal wires of a signal cable 54 by means such as welding. Further, outer peripheral portions of the cover lens 53, the first imaging device 51, the first circuit board 52, and a distal end portion of the signal cable 54 are integrally covered with insulating sealing resin or the like.

As shown in FIG. 3, the observation optical unit 28A with a super high magnification is assembled and fixed in a state that it has been inserted into and has been caused to adhere to the first hole portion 15a1 of the column-shaped member 15a. Thereby, the first imaging unit 28 where a driving temperature of the CCD of the first imaging device 51 is high is disposed inside the first hole portion 15a1 of the column-shaped member 15a. Here, the observation optical unit 28A is fixed to the first hole portion 15a1 of the column-shaped member 15a without using any fixing screw, so that a sectional area for a fixing portion of the first imaging unit 28 and the column-shaped member 15a occupied by a fixing screw can be reduced. Therefore, reduction of a diameter of the distal end portion 15a of the endoscope 2 can be made possible. Further, the first lens 41a for the first imaging unit 28 is fixed in a state that a front end portion thereof has been caused to project forward beyond a position of the flat face 25a of the projecting step portion 25.

An optical image focused on the first imaging device 51 from the first lens unit 36 is photoelectrically converted to an electric image signal by the first imaging device 51, and the image signal is output to the first circuit board 52. Further, the electric signal of the optical image output from the first circuit board 52 is transmitted to an electric device (described later) following the first circuit board 52 via the signal cable 54.

The second imaging unit 30 is configured as shown in FIG. 7. That is, the second imaging unit 30 includes a second lens unit 55 provided with a zoom optical system that can change an observation magnification from a Tele (enlargement) position to a Wide (wide angle) position continuously, and a second electric part unit 56.

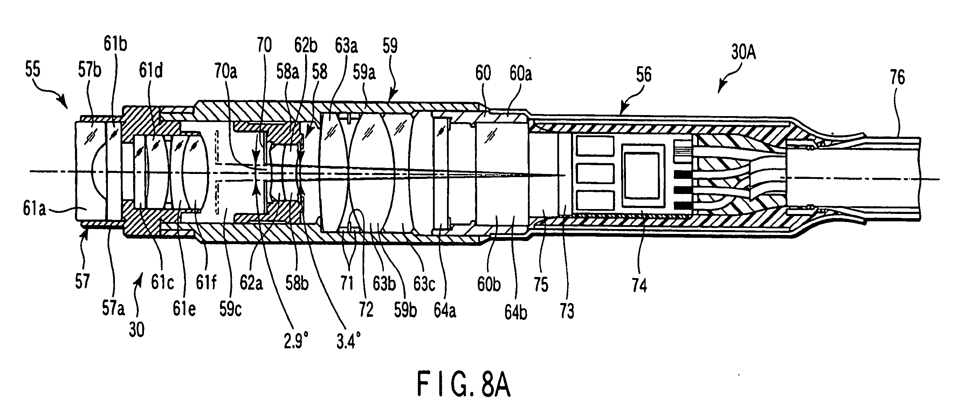

The second lens unit 55 further includes four (first to fourth) unit configuration bodies 57 to 60. The first unit configuration body 57 includes a first lens frame 57a and a first lens group 57b. As shown in FIG. 8A, the first lens group 57b includes six (first to sixth) objective lenses 61a to 61f. Here, the first lens 61a, which is an observation lens, is disposed at a distal end portion of the first lens frame 57a. For example, adhesion and fixation of the first lens 61a to the first lens frame 57a is conducted in a state that a distal end portion thereof has been caused to project forward beyond a distal end portion of the first lens frame 57a.

The second unit configuration body 58 is a moving optical unit for zooming that can advance and retreat in an photographing optical axis direction. The second unit configuration body 58 includes a second lens frame (sliding lens frame) 58a and a second lens group (zoom lens) 58b. The second lens group 58b includes two (first and second) lenses 62a and 62b.

The third unit configuration body 59 includes a third lens frame 59a and a third lens group 59b. The third lens frame 59a includes therein a guide space 59c where the second unit configuration body 58 is held to be able to advance and retreat in the photographing optical axis direction on a distal end side of the third lens frame 59a. The third lens group 59b is disposed behind the guide space 59c. The third lens group 59b includes three (first to third) lenses 63a to 63c.

The fourth unit configuration body 60 includes a fourth lens frame 60a and a fourth lens group 60b. The fourth lens group 60b includes two (first and second) lenses 64a and 64b.

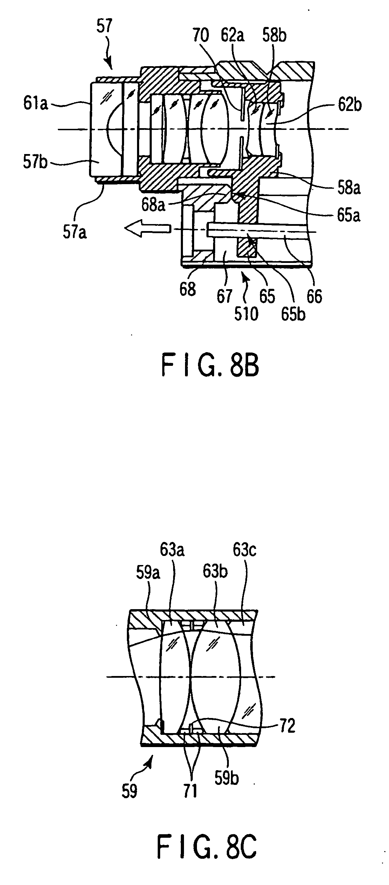

As shown in FIG. 8B, a projecting portion 65 that projects laterally is provided on one side portion of the second lens frame 58a of the second unit configuration body 58. A distal end portion of an operation wire 66 for operating the second unit configuration body 58 so as to advance and retreat in the photographing optical axis direction is fixed to the projecting portion 65.

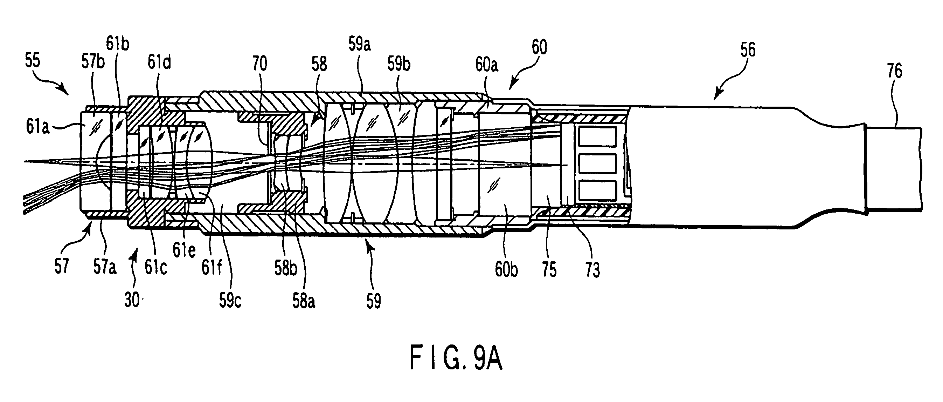

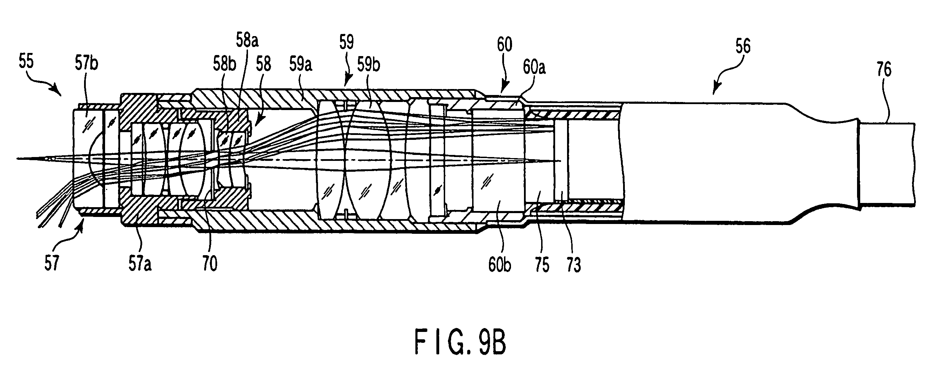

An operation lever for zooming (not shown) provided on the operation portion 12 is operated by a user so that the operation wire 66 is driven to advance and retreat in the photographing optical axis direction. At this time, the second unit configuration body 58, which is the zoom optical system, is moved forward (in a Wide (wide angle) position direction) according to such an operation that the operation wire 66 is pushed out in the distal end direction, as shown in FIG. 9B. Further, the second unit configuration body 58, which is the zoom optical system, is moved toward a near side (in Tele (enlargement) position direction) according to such an operation that the operation wire 66 is pulled in the near side direction, as shown in FIG. 9A.

A guide space 67 for the zoom guide for guiding the action of the projecting portion 65 of the second lens frame 58a moving in the zooming action direction is formed in the third lens frame 59a. A positioning member 68 for positioning of a moving end when the projecting portion 65 of the second lens frame 58a moves in the Wide (wide angle) position direction is provided at a distal end portion of the guide space 67. An abutting portion 68a that abuts on a front end portion 65a of the projecting portion 65 of the second lens frame 58a to restrict a limit position in the Wide (wide angle) position direction is formed on the positioning member 68. An abutting position where the abutting portion 68a of the positioning member 68 and the front end portion 65a of the projecting portion 65 abut on each other is disposed near a force point 65b of the projecting portion 65 of the second lens frame 58a, namely, at a position near a coupling portion between the projecting portion 65 and the operation wire 66.

Incidentally, a stopper 500 for position restriction to movement of the projecting portion 65 of the second lens frame 58a in the Tele (enlargement) side direction is provided at a rear end portion of the guide space 67. The stopper 500 is screwed and fixed to a stopper receiver 501, the maximum magnification on the Tele (enlargement) side can be adjusted by adjustment of a screwing portion.

A brightness aperture 70 is provided at the second lens frame 58a in the sliding second unit configuration body 58 for zooming, as shown in FIG. 8B. The brightness aperture 70 is disposed on a front face side of the first lens 62a held on the second lens frame 58a. The brightness aperture 70 is provided with an opening portion 70a that allows transmission of light and that is formed at a central portion of a light blocking sheet.

As shown in FIG. 8C, a plurality of (two in the present embodiment) spacer rings 71 serving as positioning members for determining a lens distance between the first lens 63a and the second lens 63b are provided in the third unit configuration body 59 in an interposing manner. A flare aperture 72 for preventing optical flare is interposed between the two spacer rings 71.

Further, a second electric part unit 56 is continuously provided at a rear end portion of the fourth unit configuration body 60. The second electric unit 56 includes a second imaging device 73 such as a CCD or a CMOS, and a second circuit board 74. Further, a cover lens 75 is provided on a light receiving side of a front face of the second imaging device 73.

The cover lens 75 of the second electric part unit 56 is fixed in a state that it is provided in parallel with an objective lens at a rear end portion of the second lens unit 55, namely, the second lens 64b of the fourth unit configuration body 60. Thereby, an observation optical unit 30A for ordinary observation obtained by integrating the second lens unit 55 and the second electric part unit 56 is formed.

The second circuit board 74 includes electric parts and a wiring pattern, and it is connected with distal end portions of a plurality of signal wires of a signal cable 76 by means such as welding. Further, outer peripheral portions of the cover lens 75, the second imaging device 73, the second circuit board 74, and a distal end portion of the signal cable 76 are integrally covered with insulating sealing resin or the like.

An optical image focused on the second imaging device 73 from the second lens unit 55 is photoelectrically converted to an electric image signal by the second imaging device 73 and the image signal is output to the second circuit board 74. Further, the electric signal of the optical image output from the second circuit board 74 is transmitted to a successive electric apparatus described later via the signal cable 76.

As shown in FIG. 3, the observation optical unit 30A for ordinary observation is assembled in a state that only the second lens unit 55 has been inserted into the third hole portion 15a3 of the column-shaped member 15a, and which is fixed by a fixing screw 77, as shown in FIG. 5A. Here, a centerline of the fixing screw 77 is disposed in a direction approximately perpendicular to an axial line direction connecting a lens center O1 of the second lens unit 55 and a wire center 02 of the operation wire 66. Thereby, the stress acting on the third lens frame 59a by the fixing screw 77 when the observation optical unit 30A is fixed to the column-shaped member 15a can be reduced so that the influence on the second unit configuration body 58 side, which is the moving optical unit for zooming, can be reduced.

Further, the second electric part unit 56 of the observation optical unit 30A is caused to project behind the third hole portion 15a3 of the column-shaped member 15a and it is disposed at a position where it does not contact the column-shaped member 15a. Thereby, since the heat of each of the two CCDs (the first imaging device 51 of the first imaging unit 28 and the second imaging device 73 of the second imaging unit 30) do not interfere with each other, heat generation of the CCDs can be suppressed. Therefore, an endoscope 2 in which noise due to heat generation of CCDs has been reduced can be obtained.

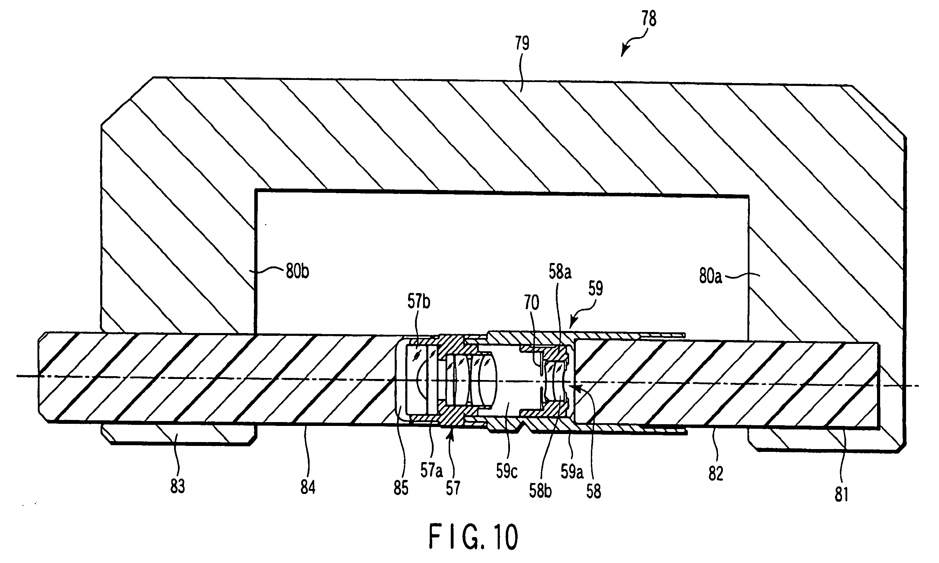

FIG. 10 shows a lens unit assembling jig 78 used at a time use of the second lens unit 55 of the second imaging unit 30. The lens unit assembling jig 78 includes an approximately U-shaped jig main body 79. The jig main body 79 includes two supporting arms 80a and 80b disposed to be spaced from and be opposed to each other.

One supporting arm 80a is formed with a fixing shaft insertion hole 81 on a side of an opposed face to the other supporting arm 80b. A fixing shaft 82 is fixed to the fixing shaft insertion hole 81 in a state that a proximal end portion thereof has been inserted into the fixing shaft insertion hole 81. A distal end portion of the fixing shaft 82 is provided to project toward the supporting arm 80b side. The distal end portion of the fixing shaft 82 can be inserted from a rear end portion of the third lens frame 59a of the third unit configuration body 59 into the lens frame 59a.

Further, a through-hole 83 provided so as to extend in the same axial direction as the fixing shaft 82 is formed on the supporting arm 80b at a position corresponding to the fixing shaft insertion hole 81 of the supporting arm 80a. A movable shaft 84 is slidably inserted into the through-hole 83. Here, accurate positioning is performed in a state that the centerline of the fixing shaft 82 and the centerline of the movable shaft 84 are arranged in the same axial line.

Further, the movable shaft 84 is provided in a projecting manner such that a distal end portion thereof is directed toward the supporting arm 80a. A lens unit insertion hole 85 in which a distal end portion of the first unit configuration body 57 of the second lens unit 55 can be inserted is formed at a distal end portion of the movable shaft 84.

At the time of assembling the second lens unit 55, the third lens frame 59a of the third unit configuration body 59 is firstly assembled to the distal end portion of the fixing shaft 82. At this time, before the first to third lenses 63a to 63c of the third lens group 59b of the third unit configuration body 59 are assembled to the third lens frame 59a, setting is performed to a state that the second unit configuration body 58 for zooming has been inserted into the third lens frame 59a in advance. Thereafter, the distal end portion of the fixing shaft 82 is inserted into the lens frame 59a from the rear end portion of the third lens frame 59a of the third unit configuration body 59. At this time, the first to third lenses 63a to 63c of the third lens group 59b of the third unit configuration body 59 have not been assembled to the third lens frame 59a. In this state, setting is performed to a state that the distal end portion of the fixing shaft 82 has been inserted from the rear end portion side of the third lens frame 59a into an assembling portion of the third lens group 59b inside the lens frame 59a.

The first unit configuration body 57 is then assembled to the distal end portion of the movable shaft 84. At this time, setting is performed to a state that the distal end portion of the first lens frame 57a of the first unit configuration body 57 has been inserted into the lens unit insertion hole 85 of the movable shaft 84.

Thereafter, the movable shaft 84 is moved toward the fixing shaft 82 side and a proximal end portion of the first lens frame 57a of the first unit configuration body 57 is inserted and fitted into the distal end portion of the third lens frame 59a. In this state, fitting portions of the proximal end portion of the first lens frame 57a and the distal end portion of the third lens frame 59a are fixed to each other by adhesive. Thereby, misalignment in optical axis among an optical axis of the first lens group 57b of the first unit configuration body 57, an optical axis of the second lens group 58b of the second unit configuration body 58, and an optical axis of the third lens group 59b assembled to the third unit configuration body 59 is corrected so that variation in assembly of the second imaging unit 30 can be prevented.

As shown in FIG. 1, the signal cable 54 of the first imaging unit 28 and the signal cable 76 of the second imaging unit 30 extend into the connector 14 via the insides of the insertion portion 11, the operation portion 12, and the universal cable 13 sequentially. A relay board 86 is incorporated with the connector 14. Proximal end portions of the signal cables 54 and 76 are connected to the relay board 86. These signal cables 54 and 76 are connected to a common cable 87 in a selectively switchable manner by the relay board 86 inside the connector 14.

Further, the relay board 86 of the connector 14 is connected to a control circuit 89, described later, inside the processor 4 via the signal cable 87 within the connector 14 and a switching signal wire 88 within the scope cable 8.

Three illumination windows, namely, the first illumination window 29, the second illumination window 31, and the third illumination window 32, disposed at the distal end portion 15 of the insertion portion 11 are provided with illumination lens units 90, respectively. As shown in FIG. 3, each illumination lens unit 90 includes a plurality of illumination lenses 91 and a holding frame 92 that holds the illumination lenses 91. Incidentally, the first illumination window 29 and the second illumination window 31 are shown in FIG. 3.

Further, the illumination lenses 91 of respective illumination lens units 90 are respectively inserted and fitted into front end portions of three hole portions, namely, the second hole portion 15a2, the fourth hole portion 15a4, and the fifth hole portion 15a5, of eight hole portions 15a1 to 15a8 formed in the column-shaped member 15a in the distal end portion 15 from their distal end sides. Here, the front end portion of the illumination lens 91 in the first illumination window 29 is fixed in a state that it has been caused to project forward beyond the position of the flat face 25a of the projecting step portion 25. Further, the front end portion of the illumination lens 91 in the first illumination window 29 is caused to project forward beyond a front end portion position of the first lens 41a for the first imaging unit 28.

Distal end portions of light guides 93 that transmit illumination lights are inserted and fitted into rear end portions of the second hole portion 15a2, the fourth hole portion 15a4, and the fifth hole portion 15a5, respectively. A distal end portion of the light guide 93 is capped with a cylindrical member 94, and it is covered with an outer skin 95 bundling a plurality of fibers and a protective tube 502 that is a rubber material.

The light guide 93 extends into the connector 14 via insides of the insertion portion 11, the operation portion 12, and the universal cable 13 sequentially. A proximal end portion 96 side of the light guide 93 is connected to a light guide connector (not shown) projecting from the connector 14. The light guide connector is attachably and detachably connected to the light source apparatus 3.

The light source apparatus 3 includes a lamp 97 generating white light, a collimator lens 98 collimating light from the lamp 97 to a beam, and a converging lens 100 focusing transmission light from the collimator lens 98 to output the same to the proximal end portion 96 of the light guide 93. Incidentally, the light source apparatus 3 includes a light adjusting function (not shown) for adjusting the brightness of illumination light of the lamp 97.

In the present embodiment, the light guide 93 is branched, for example, inside the operation portion 12 and inserted through the insertion portion 11 in a state that it has been divided into three pieces. Distal end portions of three branched light guides 93 are respectively disposed near rear faces of the respective illumination lenses 91 in three illumination windows, namely, the first illumination window 29, the second illumination window 31, and the third illumination window 32, provided in the distal end cover 24 to be opposed thereto, and they are fixed to rear end portions of the second hole portion 15a2, the fourth hole portion 15a4, and the fifth hole portion 15a5 in the column-shaped member 15a by, for example, setscrews.

Illumination light from the lamp 97 in the light source apparatus 3 is emitted on the distal end portion 96 of the light guide 93, and illumination light guided through the light guide 93 is emitted forward beyond the endoscope 2 via the respective illumination lenses 91 of the first illumination window 29, the second illumination window 31, and the third illumination window 32.

As shown in FIG. 4A, a distal end portion of a communication pipe 105 communicating with the procedure tool insertion channel 33 is inserted and fitted into the sixth hole portion 15a6 formed in the column-shaped member 15a at the distal end portion 15 from the proximal end portion side. A proximal end portion of the communication pipe 105 is caused to project rearward behind the column-shaped member 15a and a distal end portion of the procedure tool insertion channel 33 is coupled to the proximal end portion of the communication pipe 105. A distal end of the procedure tool insertion channel 33 is caused to communicate with the distal end opening portion 33a of the distal end cover 24.

The procedure tool insertion channel 33 is branched near the distal end of the insertion portion 11 and one of branched channels is inserted up to a procedure tool insertion port (not shown) disposed in the operation portion 12. The other of the branched channels communicates with a suction channel through insides of the insertion portion 11 and the universal cable 13, and a proximal end thereof is connected to suction means (not shown) via the connector 14.

As shown in FIG. 6, a proximal end portion of the air-supplying/water-supplying nozzle 34 is inserted and fitted into a front end portion of the seventh hole portion 15a7 formed in the column-shaped member 15a in the distal end portion 15. Further, a distal end portion of a communication pipe 107 communicating with the air-supplying/water-supplying pipe conduit 106 for the air-supplying/water-supplying nozzle 34 is inserted and fitted into a rear end portion of the seventh hole portion 15a7. A proximal end portion of the communicating pipe 107 is caused to project rearward behind the column-shaped member 15a, and a distal end portion of the air-supplying/water-supplying pipe conduit 106 is coupled to a proximal end portion of the communication pipe 107. Incidentally, the communication pipe 107 and the air-supplying/water-supplying pipe conduit 106 are connected and fixed to each other by a spool.

A proximal end portion of the air-supplying/water-supplying pipe conduit 106 is coupled to a branch pipe 108. Here, branched end portions 108a and 108b of the branch pipe 108 are connected with distal end portions of an air-supplying pipe conduit 106a and a water-supplying pipe conduit 106b, respectively. Thereby, the air-supplying/water-supplying pipe conduit 106 communicates with the air-supplying pipe conduit 106a and the water-supplying pipe conduit 106b. Incidentally, the respective pipe conduits 106, 106a, and 106b, and the branch pipe 108 are connected and fixed to each other by spools, and, for example, adhesive or the like is applied to peripheral portions of respective connection portions and the whole branch pipe 108 so that the respective connection portions are kept air-tight (water-tight).

The air-supplying pipe conduit 106a and the water-supplying pipe conduit 106b communicating with the air-supplying/water-supplying nozzle 34 are inserted up to the connector 14 of the universal cable 13, and it is connected to the air-supplying/water-supplying apparatus 6 incorporated with a pump (not shown) conducting air supply and water supply.

An air-supplying/water-supplying button 109 disposed on the operation portion 12 is arranged in intermediate portions of the air-supplying pipe conduit 106a and the water-supplying conduit 106b. Air supply and water supply are performed when the air-supplying/water-supplying button 109 is operated.

Thereby, gas such as air or liquid such as sterile water is jetted from a jetting port 34a of the air-supplying/water-supplying nozzle 34 in a jetting direction. At this time, fluid such as sterile water or air jetted from the jetting port 34a of the air-supplying/water-supplying nozzle 34 is guided toward the first lens 61a side for the second imaging unit 30 along the fluid guide face 26c to clean body fluid or dirt such as extraneous matter which has adhered to the surface of the first lens 61a for the second imaging unit 30, so that the imaging and observation field of view can be maintained in a clean state.

FIG. 13 shows a configuration of the pipe conduit 35 for forward water supply having the opening 35a on the distal end cover 24. As shown in FIG. 13, a distal end portion of an approximately cylindrical pipe member 35b is inserted and fitted into the eighth hole portion 15a8 formed at the column-shaped member 15a at the distal end portion 15 from a rear end portion side of the pipe member 35b. A proximal end portion of the pipe member 35b is caused to project rearward behind the column-shaped member 15a and a distal end portion of the pipe conduit 35 for forward water supply is coupled to the proximal end portion of the pipe member 35b. Incidentally, the distal end portion of the pipe conduit 35 for forward water supply covers the proximal end portion of the pipe member 35b, and the distal end portion is connected and fixed to the proximal end portion by a spool.

The pipe conduit 35 for forward water supply is inserted up to the connector 14 through the insertion portion 11, the operation portion 12, and the universal cable 13 to be connected to the forward water-supplying apparatus 7. A forward water-supplying button (not shown) is disposed in an intermediate portion of the pipe conduit 35 for forward water supply on the operation portion 12.

When the forward water-supplying button is operated, a liquid, such as sterile water, is sprayed from the opening portion 35a of the distal end cover 24 of the insertion portion 11 in an insertion direction to a body cavity. Thereby, body fluid that has adhered to a site to be examined inside the body cavity or the like can by removed. Incidentally, as shown in FIG. 1, a foot switch 7a is connected to a cable extending from the forward water-supplying apparatus 7, and a user can also spray a liquid such as sterile water from the distal end face of the insertion portion 11 in the insertion direction to the body cavity by the operation of the foot switch 7a.

Furthermore, as shown in FIG. 16A, at a corner portion end edge portion on an opposite side spaced from an arrangement position of the first lens 41a on the flat face 25a of the projecting step portion 25, an outer peripheral face of the distal end cover 24 of the insertion portion 11 has a chamfer angle enlargement portion 202 (shown by a solid line in FIG. 16) having a chamfer angle larger than that of a corner portion end edge portion 201 on a side face other than the corner portion end edge portion 201 on the opposite side. The chamfer angle enlargement portion 202 and the corner portion end edge portion 201 on the side face other than the chamfer angle enlargement portion 202 are set to have a chamfer angle R of 1 to 1.3 mm or so and a chamfer angle R of 0.7 to 1 mm or so, respectively.

Drive circuits 110a and 110b for driving the first imaging device 51 in the first imaging unit 28 and the second imaging device 73 in the second imaging unit 30, respectively, a signal processing circuit 111 for performing signal processing on imaging signals respectively output from the two imaging devices 51 and 73 via the relay board 86, and a control circuit 89 for controlling action states of the signal processing circuit 111 and the like are provided inside the processor 4.

Control switches 112a and 112b, the air-supplying/water-supplying button 109, the bending operation knobs (not shown), a zoom lever (not shown) for performing zoom operation of the second imaging unit 30 for ordinary observation, the forward water-supplying button (not shown), and the abovementioned procedure tool insertion port (not shown) are provided on the operation portion 12 of the endoscope 2.

These control switches 112a and 112b are connected to the control circuit 89 in the processor 4 through signal wires 113a and 113b, respectively. In the present embodiment, for example, the control switch 112a generates a signal for instructing switching, while the control switch 112b generates, for example, a signal for freeze instruction.

The relay board 86 conducts a switching action from such a state that one of the signal cables 54 and 76 connected to the respective imaging devices 51 and 73 has been connected to a common signal cable 87 to such a state that the other signal cable is connected to the signal cable 87, for example, according to operation of the control switch 112a.

Specifically, for example, a switching signal is output from the control circuit 89 to the relay board 86 via the switching signal wire 88 in the scope cable 8 by operating the control switch 112a. The relay board 86 is put in a L (LOW) level state when an input end of a signal from the control circuit 89 is normal, where a switching control terminal is pulled down. In this state, the signal cable 54 of the second imaging unit 30 for ordinary observation is connected to the common signal cable 87. Even in an actuation start state, the switching control terminal takes the L level. That is, unless an operation for a switching instruction is conducted, setting to an ordinary observation state is maintained.

In this state, when a user operates the control switch 112a, a signal from the control circuit 89 is applied to an input end of the relay board 86 via the switching signal wire 88 as a control signal changing to the H (HIGH) level, so that the switching control terminal is pulled up. In this state, the signal cable 54 of the first imaging unit 28 of an object-contacting type is connected to the common signal cable 87.

Further, when the control switch 112a is operated, a signal of the L level is supplied to the switching control terminal, so that the signal cable 54 of the second imaging unit 30 for ordinary observation is connected to the common signal cable 87.

The control circuit 89 conducts control such that an action state of the signal processing circuit 111 is conducted corresponding to either of the imaging device 51 of the second imaging unit 30 for ordinary observation and the imaging device 73 of the first imaging unit 28 of an object-contacting type according to operation of the control switch 112a.

Each endoscope image of the first imaging unit 28 of an object-contacting type and the second imaging unit 30 for ordinary observation is displayed on the monitor 5 by input of a video signal output from the signal processing circuit 111 in the processor 4.

Subject images photographed by the respective two imaging units 28 and 30 are displayed on the monitor 5 (see FIG. 1). Here, upward and downward directions of the monitor 5 coincide with a vertical transfer direction of the CCD device or the CMOS device of each imaging device 51, 73, while leftward and rightward directions coincide with a horizontal transfer direction of the CCD device or the CMOS device of each imaging device 51, 73. That is, upward, downward, leftward, and rightward directions of the endoscope images photographed by the respective two imaging units 28 and 30 coincide with upward, downward, leftward, and rightward directions on the monitor 5.

Upward, downward, leftward, and rightward directions of the bending portion 16 of the insertion portion 11 are determined so as to correspond to upward, downward, leftward, and rightward directions of the endoscope image displayed on the monitor 5. That is, four bending operation wires 23 inserted in the bending portion 16 are pulled and loosened by predetermined operations of the bending operation knobs provided on the operation portion 12 so that the bending portion 16 is freely bent in the four directions of the upward, downward, leftward, and rightward directions corresponding to the upward, downward, leftward, and rightward directions of an image displayed on the monitor 5.

That is, arrangement directions of the two imaging units 28 and 30 within the distal end portion 15 are determined such that horizontal transfer directions and vertical transfer directions of the respective imaging devices 51 and 73 correspond to each other respectively. Thereby, even if ordinary observation and enlargement observation of an object-contacting type are switched from one to the other, an endoscope image displayed on the monitor 5 is maintained such that upward, downward, leftward, and rightward directions corresponding to the bending operation direction of the bending portion 16 are always constant. As a result, a user can conduct bending operations in upward, downward, leftward, and rightward directions of the bending portion 16 without feeling disoriented regarding upward, downward, leftward, and rightward directions of an endoscope image displayed on the monitor 5 when the endoscope image has been switched between an ordinary observation image and an enlargement observation image.

Next, operation of the endoscope system 1 with the abovementioned configuration will be explained. The endoscope system 1 is set during use of the endoscope 2 according to the present embodiment as shown in FIG. 1. That is, a user connects the connector 14 of the endoscope 2 to the light source apparatus 3, one end of the scope cable 8 to the connector 14, and the other end of the scope cable 8 to the processor 4. The user connects the air-supplying pipe conduit 106a and the water-supplying pipe conduit 106b to the air-supplying/water-supplying apparatus 6.

The user turns ON power sources of the light source apparatus 3, the processor 4, and the like to set these devices in operating states. At this time, the control circuit 89 in the processor 4 can transmit and receive control signals and the like.

In the actuated sate, the relay substrate 86 is set such that the second imaging unit 30 side for ordinary observation is selected. At this time, the control circuit 89 performs control so as to drive the drive circuit 110b and set the operating state of the signal processing circuit 111 to an observation mode for ordinary observation.

After setting of the endoscope system 1 is completed, a work for inserting the endoscope 2 into a patient's body is started. The user inserts the insertion portion 11 of the endoscope 2 into a body cavity and conducts setting so as to be capable of observing an affected site to be diagnosed or the like during insertion work of the endoscope 2.

The light source apparatus 3 is put in a supplying state of illumination light. For example, illumination light of RGB is supplied to the light guide 93 in a frame sequential manner. In synchronism therewith, the drive circuit 110b outputs a CCD drive signal and illuminates an affected site in a body cavity of the patient via the first illumination window 29 and the second and third illumination windows 31 and 32.

A subject such an affected site illuminated is focused on a light receiving face of the second imaging device 73 through the second lens unit 55 of the second imaging unit 30 for ordinary observation and photoelectrically converted. The second imaging device 73 outputs the photoelectrically-converted signal according to application of the drive signal. The signal is input into the signal processing circuit 111 via the signal cable 76 and the common signal cable 87 selected by the relay substrate 86. After the signal inputted into the signal processing circuit 111 is A/D-converted inside the circuit 111, it is temporarily stored in the memory for R, G, and B.

Thereafter, the signals stored in the memory for R, G, and B are simultaneously read and are changed to synchronized R, G, and B signals, and are then further D/A converted to change to analog R; G, and B signals, so that the signals are displayed on the monitor 5 in a color display. Thereby, ordinary observation of an object to be observed which is spaced from the first lens 61a for the second imaging unit 30 can be performed in a wide range using the second imaging device 30 for ordinary observation.

When matter such as body fluid or extraneous matter adheres to a surface of the first lens 61a of the second imaging unit 30 during ordinary observation, the air-supplying/water-supplying button 109 is operated. Air supply and water supply are conducted through the air supply pipe conduit 106a and the water supply pipe conduit 106b by the operation of the air-supplying/water-supplying button 109. Gas such as air or liquid such as sterile water is jetted in a jetting direction from the jetting port 34a of the air-supplying/water-supplying nozzle 34 at the low step portion 27 of the projecting step portion 25. At this time, fluid such as sterile water or air jetted from the jetting port 34a of the air-supplying/water-supplying nozzle 34 is guided to the first lens 61a side for the second imaging unit 30 along the fluid guide face 26c of the projecting step portion 25 and matter such as body fluid or extraneous matter which has adhered to a surface of the first lens 61a of the second imaging unit 30 is removed and cleaned so that a clear image and observation field of view can be secured.

Further, when a site to be examined in a body cavity has become adhered with body fluid or the like, the forward water-supplying button is operated. Liquid such as sterile water is sprayed from the opening portion 35a of the distal end cover 24 of the insertion portion 11 in an insertion direction to the body cavity at a time of operation of the forward water-supplying button. Thereby, the body fluid which has adhered to the site to be examined in the body cavity or the like can be cleaned.

Observation conducted by the second imaging unit 30 for ordinary observation is continued until a distal end portion of the endoscope 2 inserted into the body of the patient is guided up to a targeted site to be observed. The control switch 112a is turned ON in a state that the distal end portion of the endoscope 2 comes close to the targeted site to be observed.

The control circuit 89 receives a switching instruction signal to conduct switching control of the relay substrate 86 at a time of ON operation of the control switch 112a. At this time, the control circuit 89 controls the drive circuit 110b to an operating state and sets the signal processing circuit 111 to an observation mode with a high magnification. Thereby, switching is performed from the mode for ordinary observation conducted by the second imaging unit 30 to an observation mode with a high magnification using the first imaging unit 28.

Thus, object-contacting observation with a high magnification or the like can be performed in a state that switching to the observation mode with a high magnification has been conducted. In the object-contacting observation with a high magnification, a cell tissue to be observed or the like is observed with a high magnification while a distal end of the first lens 41a for the first imaging unit 28 is in contact with the object to be observed. The first imaging unit 28 is configured so as to be capable of observing an object to be observed with a magnification of a histogenetical magnification level where a structure of a body tissue including a cell or a structure of a gland duct is observed and it has a magnification in a range of about 200 times to about 1,000 times. Incidentally, when enlarged observation is performed with a high magnification, for example, dye is sprayed on a site of interest in advance and the site of interest is stained so that a contour of a cell can be observed sharply.

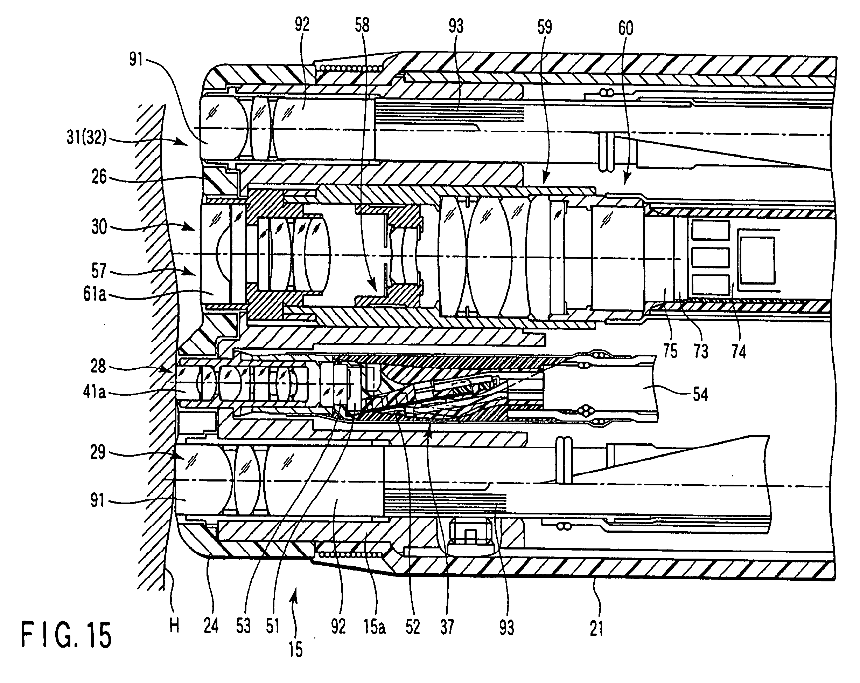

The distal end portion 15 of the insertion portion 11 is pressed on a surface of a body tissue H during observation of the body tissue H conducted by the first imaging unit 28 of an object-contacting type. At this time, as shown in FIG. 15, the projecting step portion 25 of the distal end cover 24 is mainly pressed on the surface of the body tissue H, while a non-projecting face other than the projecting step portion 25 is kept in a contact state with the surface of the body tissue H. Therefore, the first lens 41a at the distal end of the first imaging unit 28 and the illumination lens 91 in the first illumination window 29 disposed in the projecting step portion 25 are brought in contact with the surface of the body tissue H such as a cell tissue to be observed. Incidentally, an observation range of the first imaging unit 28 with a super-high magnification is shallow in observation depth, such as 0 to 100 $\mu$m, from the first lens 41a, which is the observation window, which may result in an unstable observation state due to shake or focus error (incidentally, here the observation depth of 0 $\mu$m means that a focus position of the first imaging unit 28 lies on the surface of the first lens 41a, which is the observation window). Therefore, when observation is performed using the first imaging unit 28 with a super-high magnification, observation is performed in a state that the first lens 41a, which is the observation window, is brought in contact with an object to be examined and the distal end portion 15 of the endoscope is maintained in an immovable state. Since the observation depth and an observation magnification are generally in an inversely proportional relationship, the observation depth becomes shallow according to the increase in optical magnification. More specifically, when the optical magnification is increased to about 1,000 times, the observation depth is generally reduced to 0 to about 5 $\mu$m.

In this state, illumination light is emitted on a body tissue H such as a cell tissue through the illumination lens 91 of the first illumination window 29. At this time, a portion of illumination light emitted on the body tissue H such as a cell tissue is caused to transmit through the body tissue H such as a cell tissue, as shown by an arrow in FIG. 16A, and is diffused around an abutting face of the illumination lens 91 of the first illumination window 29. Therefore, the illumination light is emitted on a peripheral part of a body tissue H such as cell tissue in front of the first lens 41a of the first imaging unit 28. Thereby, illumination light is also irradiated on a portion to be observed by the first lens 41a for the first imaging unit 28 pressed on the surface of the body tissue H such as a cell tissue, so that light from the body tissue H such as a cell tissue passes through the lens unit 36 in the first imaging unit 28 to be focused on the light receiving face of the first imaging device 51 to be photoelectrically converted.

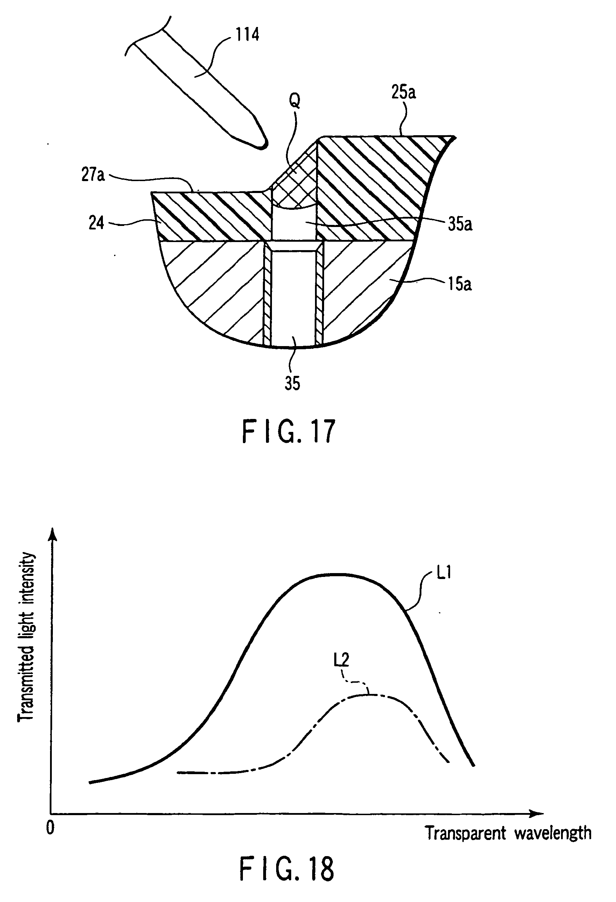

Incidentally, in FIG. 16A, reference symbol 03 denotes a center position of the first lens 41a for the first imaging unit 28, 04 denotes a center position of the illumination lens 91 in the first illumination window 29, and L denotes a distance between the center position 03 of the first lens 41a and the center position 04 of the illumination lens 91. Further, FIG. 18 is a characteristic diagram showing a relationship between transmitted light intensity and transparent wavelength for explaining a difference in observation state due to a difference in distance L between the center position 03 of the first lens 41a for the first imaging unit 28 and the center position 04 of the illumination lens 91 in the first illumination window 29 during observation conducted by the first imaging unit 28 of a object-contacting type. Here, L1 < L2. As also apparent from the characteristic diagram shown in FIG. 18, when the distance L between the center position 03 of the first lens 41a and the center position 04 of the illumination lens 91 is short (L1), the transmitted light intensity becomes large. Further, it is found that light on the side of a short wavelength is attenuated more easily than light on the side of a long wavelength due to scattering of light in the body tissue.

The first imaging device 51 outputs a photoelectrically-converted signal by application of a drive signal from the drive circuit 110b. In this case, the signal is amplified inside the first imaging device 51 and output from the first imaging device 51. The signal is input into the signal processing circuit 111 via the signal cable 54 and the common signal cable 87 selected by the relay substrate 86.

After the signal input into the signal processing circuit 111 is A/D-converted to R, G, and B signals internally, the R, G, and B signals are simultaneously stored in the memory for R, G, and B signals. The signals stored in the memory for R, G, and B signals are simultaneously read, are changed to synchronized R, G, and B signals, and are further D/A-converted to analog R, G, B signals to be displayed on the monitor 5. Thereby, observation of a body tissue H such as a cell tissue in front of the first lens 41a of the first imaging unit 28 is performed in an observation mode with a high magnification using the first imaging unit 28 of an object-contacting type.

With the abovementioned configuration, the following effects can be obtained. That is, according to the present embodiment, the air-supplying/water-supplying nozzle 34 that deliveries fluid to the first lens 61a, which is the observation lens for the second imaging unit 30, is disposed at the low step portion 27 in the distal end portion 15 of the insertion portion 11. As a result, the height of the air-supplying/water-supplying nozzle 34 can be lowered. Thereby, when observation is conducted in a state that the flat face 25a of the projecting step portion 25 in the distal end portion 15 of the insertion portion 11 is in contact with a body tissue (a body to be examined), the possibility can be reduced that the air-supplying/water-supplying nozzle 34 is caught by a living body. Therefore, such a possibility can be reduced that the jetting port 34a of the distal end opening portion of the air-supplying/water-supplying nozzle 34 is caught by an object to be observed and such a possibility can be decreased that a surface of a body tissue positioned at a site to be observed is injured, so that observation or diagnosis work utilizing the first imaging unit 28 of an object-contacting type can be conducted easily.

Further, in the present embodiment, as shown in FIG. 12, since the distal end face of the jetting port 34a of the distal end opening portion of the air-supplying/water-supplying nozzle 34 and the first lens 61a, which is the observation lens for the second imaging unit 30, are disposed so as to be approximately flush with each other, such an effect that an excellent draining property is achieved can be obtained at a cleaning time.

As shown in FIG. 12, the jetting port 34a of the distal end opening portion of the air-supplying/water-supplying nozzle 34 is disposed behind the projecting face, which is the flat face 25a of the projecting step portion 25. Therefore, when the flat face 25a of the projecting step portion 25 is caused to abut on a body to be examined, the distal end portion of the air-supplying/water-supplying nozzle 34 can be reliably prevented from being caught by the body to be examined.

As shown in FIG. 16A, a chamfer angle enlargement portion 202 with a chamfer angle larger than that of a corner portion end edge portion 201 on a side face other than the chamfer angle enlargement portion 202 is provided at an opposite side corner portion end edge portion spaced from an arrangement position of the first lens 41a on the flat portion 25a of the projecting step portion 25 on an outer peripheral face of the distal end cover 24 of the insertion portion 11. Thereby, when the flat face 25a of the projecting step portion 25 is caused to abut on a portion to be examined, as shown in FIG. 16A, the opposite side portion spaced from the arrangement position of the first lens 41a of the projecting step portion 25 can be brought in contact with the portion to be examined more easily as if a portion with a large chamfer angle such as the chamfer angle enlargement portion 202 shown by a solid line in FIG. 16A could be brought in contact with a portion to be examined more easily than a portion with a small chamfer angle such as the corner portion end edge portion 201 (shown by an imaginary line in FIG. 16A) on the side face other than the chamfer angle enlargement portion 202. Therefore, when observation is performed utilizing the first imaging unit 28 of an object-contacting type, the first lens 41a of the projecting step portion 25 can be caused to abut on a

body to be examined.

Setting is performed such that the chamfer angle enlargement portion 202 at the distal end cover 24 of the insertion portion 11 has a chamfer angle R of 1 to 1.3 mm or so and the corner portion end edge portion 201 on the side face except for the chamfer angle enlargement portion 202 has a chamfer angle R of 0.7 to 1 mm or so. Therefore, since it is unnecessary to increase of the chamfer angle R of the chamfer angle enlargement portion 202 of the distal end cover 24 of the insertion portion 11 beyond necessity, such an effect that the distal end portion 15 of the endoscope 2 does not become thick can be obtained.

The endoscope 2 according to the present embodiment has various features (effects) owing the structure explained below. As shown in FIG. 2 and FIG. 3, first, the first lens 41a in the observation window of the first imaging unit 28 with a super-high magnification is disposed on the flat face 25a of the projecting step portion 25 at the distal end portion 15 of the endoscope 2. Here, since the first imaging unit 28 with a super-high magnification has a narrow observation depth, such an effect can be achieved that, when observation with a super-high magnification is performed in a contact state with a body tissue (a body to be examined) H, approach to a body tissue is facilitated by providing the first imaging unit 28 with a super-high magnification on the flat face 25a of the projecting step portion 25.

The first imaging unit 28 with a super-high magnification performing contact observation is arranged in a direction in which an operator can operate the first imaging unit 28 easily, that is, a center of the first imaging unit 28 (with a super-high magnification) is arranged on a vertical center axis of the second imaging unit 30 for ordinary observation in an endoscope 2 having at least two imaging units. Thereby, when a plurality of observation windows is provided, parallax occurs at a time of observation, but approach easiness to a body tissue (a body to be examined) is improved by arranging a plurality of observation windows (the first lens 41a for the first imaging unit 28 and the first lens 61a for the second imaging unit 30) in a direction in which the windows can be easily operated by an operator.

Further, two angle knobs (for a vertical direction and a horizontal direction) are arranged on the operation portion of the endoscope 2, but since the angle knob for a vertical direction can be operated by the thumb of an operator, such an effect can be obtained that the operator can easily work and approach easiness to a body tissue is also improved at a time of observation with a super-high magnification.

As shown in FIG. 2, the opening portion 35a for forward water supplying is provided near the vertical center axis of the second imaging unit 30 for ordinary observation. Here, an operation in combination with an operation of the angle knob for the vertical direction is frequently conducted when body fluid or the like that has adhered to a site to be examined in a body cavity is cleaned by conducting forward water supply from the opening portion 35a for forward water supplying. Therefore, since the angle knob for a vertical direction mainly used can be operated by the thumb, such an effect can be achieved that the operator can work easily, workability for forward water supplying can be improved, and approach easiness to a body tissue at a time of observation with a super-high magnification can be improved.

The projecting step portion 25 at the distal end portion 15 of the endoscope 2 where the first imaging unit 28 with a super-high magnification and the first lens 41a, which is the illumination window for an imaging unit, is disposed on an approximately right lower side (an approximately left lower side to FIG. 2) on the display monitor to the second imaging unit 30 for ordinary observation. In general, an operator can conduct rightward twisting of the insertion portion 11 more easily than leftward twisting thereto at a time of operation. Therefore, such an effect can be obtained that approach easiness to a body tissue can be improved at a time of observation conducted by the first imaging unit 28 with a super-high magnification by arranging a contacting portion with a body tissue (a body to be examined) H on a right lower side on the second imaging unit 30 for ordinary observation.

The distal end opening portion 33a of the procedure tool insertion channel 33 is arranged near an approximately left lower side to the first lens 41a of the observation window of the first imaging unit 28 with a super-high magnification. Thereby, a body tissue (a body to be examined) H can be pulled toward the distal end of the insertion portion 11 by conducting suction from the distal end opening portion 33a of the procedure tool insertion channel 33, so that further stabilized observation utilizing the first imaging unit 28 with a super-high magnification can be made possible.

Furthermore, since it is necessary to arrange the first lens 41a of the observation window of the first imaging unit 28 with a super-high magnification on a right lower side to the second imaging unit 30 for ordinary observation, an internal space within the insertion portion 11 can be used efficiently by arranging the procedure tool insertion channel 33 on the left lower side, which is a dead space, so that the insertion portion 11 can be made slender.

The fluid guide face 26c with a gentle slope angle (angularity of about 18°) is disposed between the air-supplying/water-supplying nozzle 34 of the low step portion 27 at the distal end portion 15 of the insertion portion 11 and the second imaging unit 30 of the middle step portion 26, and the other inclined face of the wall portion between the lower step portion 27 and the middle step portion 26 is formed at an angle of about 45°. Thereby, delivery water jetted from the jetting port 34a of the distal end opening portion of the air-supplying/water-supplying nozzle 34 can be caused to flow along an extension line of the nozzle jetting port 34a in a rectified state so that delivery of water is improved. As a result, improvement of securing of field of view for ordinary observation using the second imaging unit 30 can be achieved.

As shown in FIGS. 5A and 5B, a zoom sliding section mechanism for zooming of the second imaging unit 30 is disposed in a dead space of the fluid guide face 26c that guides delivery water jetted from the jetting port 34a of the air-supplying/water-supplying nozzle 34 to the first lens 61a for the second imaging unit 30 along the extension line of the nozzle jetting port 34a. Here, when the projecting step portion 25 at the distal end portion 15 of the insertion portion 11 is made high, it is necessary to set the slope angle of the fluid guide face 26c at the jetting port 34a of the air-supplying/water-supplying nozzle 34 to about 18° in view of the draining property, which results in enlargement of the dead space in the insertion portion 11. Therefore, the diameter of the endoscope insertion portion 11 can be reduced by arranging the zoom sliding portion mechanism 510 mounted on the second imaging unit 30 for ordinary observation in the dead space of the fluid guide face 26c.

Further, in the present embodiment, an air-supplying/water-supplying nozzle is not provided on the first lens 41a which is the observation lens for the first imaging unit 28 utilized for contact observation to a body tissue (a body to be examined), and only the air-supplying/water-supplying nozzle 34 for cleaning the first lens 61a which is the observation lens for the second imaging unit 30 for ordinary observation except for the first imaging unit 28 is provided. Here, even if mucus or residue adheres to the first lens 41a for the first imaging unit 28 contacting with a body tissue (a body to be examined), it can be removed at a time of contacting the body tissue (a body to be examined), so that it is unnecessary to provide an air-supplying/water-supplying nozzle. Therefore, the number of nozzles 34 to be provided can be reduced to the minimum by providing the air-supplying/water-supplying nozzle 34 for cleaning only the first lens 61a for the second imaging unit 30 for ordinary observation so that the diameter of the endoscope insertion portion 11 can be reduced.

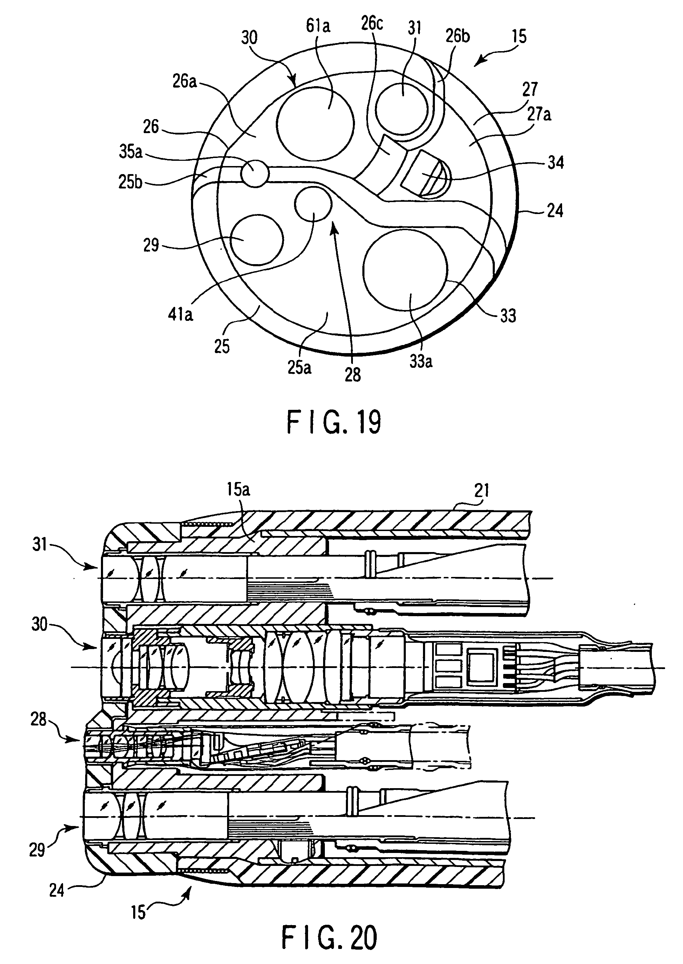

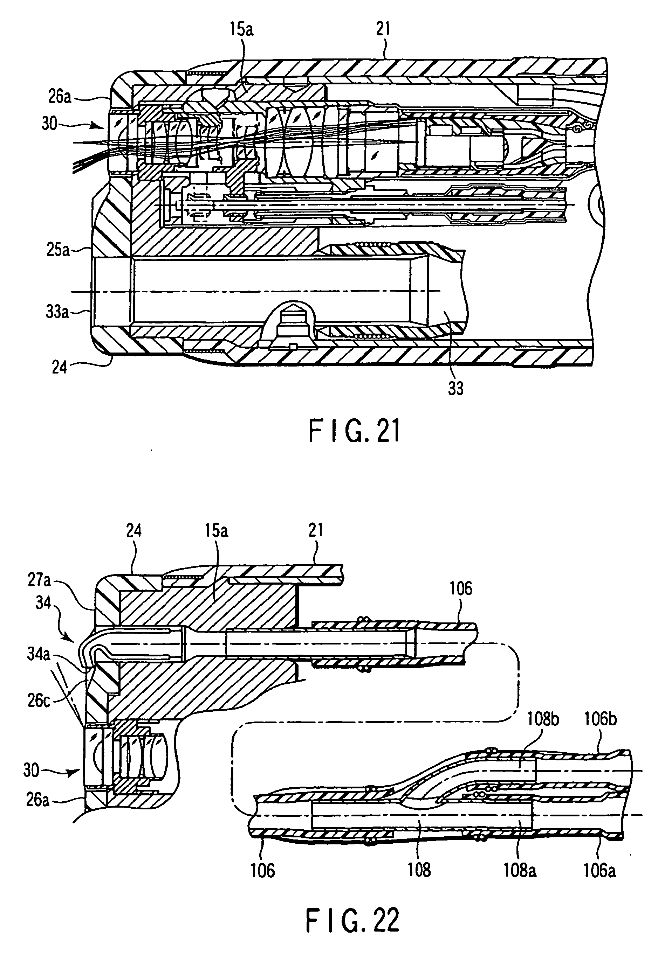

FIGS. 19 to 22 show a second embodiment of the present invention. The present embodiment has a configuration where the configuration of the endoscope 2 of the endoscope system 1 according to the first embodiment (see FIGS. 1 to 18) has been changed in the following manner.

That is, in an endoscope 2 according to the present embodiment, an area of a flat face 25a of a projecting step portion 25 contacting with an object is made larger than that of the flat face 25a according to the first embodiment, as shown in FIG. 19. The flat face 25a of the projecting step portion 25 of the distal end cover 24 is formed on an approximately lower half portion of the whole circular front face of the distal end cover 24 to have an area of about 1/2 of the whole circular front face of the distal end cover 24.

Further, a first lens 41a, which is an observation lens for a first imaging unit (a first observation portion) 28 of an object-contacting type, a first illumination window 29, and a distal end opening portion 33a of a procedure tool insertion channel 33 are arranged on the flat face 25a of the projecting step portion 25 according to the present embodiment.

Further, a flat face 26a of a middle step portion 26 that is lower than the projecting step portion 25 by one step is formed on an approximately upper half portion of the whole front face of the distal end cover 24 on a left side portion in FIG. 19 and a flat face 27a of a lower step portion 27 that is lower than the middle step portion 26 by one step is formed on a right side portion in FIG. 19. Here, the middle step portion 26 occupies an area of approximately 2/3 of the upper half portion of the distal end cover 24, while the low step portion 27 has the smallest area.

A first lens 61a, which is an observation lens for a second imaging unit (second observation section) 30 for ordinary observation, and one (second) illumination window 31 are disposed on the flat face 26a of the middle step portion 26. Here, the first lens 61a for the second imaging unit 30 is disposed at a position just above a first lens 41a of a first imaging unit 28. A second illumination window 31 is disposed on the right side of the first lens 61a of the second imaging unit 30. Further, an air-supplying/water-supplying nozzle 34 is disposed on the flat face 27a of the low step portion 27.

Further, an inclined face 25b with an inclination angle of, for example, about 45° is formed on a wall portion between the projecting step portion 25, and the middle step portion 26 and the low step portion 27. An opening portion 35a for forward water supply is disposed on the inclined face 25b.

Moreover, an inclined face 26b with an inclination angle of, for example, about 45° and a fluid guide face 26c with an inclination angle smaller than that of the inclined face 26b are formed on a wall portion between the middle step portion 26 and the low step portion 27.

The fluid guide face 26c is disposed between the air-supplying/water-supplying nozzle 34 on the low step portion 27 and the second imaging unit 30 on the middle step portion 26. The fluid guide face 26c is formed by a gentle inclined face with an inclination angle of, for example, about 18°. Incidentally, the endoscope according to the second embodiment has the same configuration as that of the endoscope 2 according to the first embodiment except for the abovementioned configuration. The same portions or members as those of the endoscope 2 according to the first embodiment are attached with same reference numerals, and explanation thereof is omitted.

In the endoscope 2 according to the present embodiment, the second imaging unit 30 for ordinary observation is used at a time of insertion work for inserting the endoscope 2 into a body of a patient, as in the first embodiment. Thereby, ordinary observation for observing an object to be observed which is spaced from the first lens 61a of the second imaging unit 30 over a wide range is performed using the second imaging unit 30 for ordinary observation.

When matter such as body fluid or extraneous matter adheres to a surface of the first lens 61a for the second imaging unit 30 during this ordinary observation, the air-supplying/water-supplying button 109 is operated. Air supply and water supply are conducted through the air-supplying pipe conduit 106a and the water-supplying pipe conduit 106b according to operation of the air-supplying/water-supplying button 109. A gas such as air or liquid such as sterile water is jetted in a jetting direction from the jetting port 34a of the air-supplying/water-supplying nozzle 34 on the low step portion 27 of the projecting step portion 25. At this time, a fluid such as sterile water or air jetted from the jetting port 34a of the air-supplying/water-supplying nozzle 34 is guided toward the first lens 61a side for the second imaging unit 30 along the fluid guide face 26c of the projecting step portion 25 so that matter such as body fluid or extraneous matter which has adhered to the surface of the first lens 61a for the second imaging unit 30 is removed and cleaned, and imaging and the observation field of view in a clean state can be assured.

Further, when body fluid adheres to a site to be examined in a body cavity, which soils the site, the forward water-supplying button is operated. A liquid such as sterile water is sprayed from the opening portion 35a of the distal end cover 24 of the insertion portion 11 in an insertion direction toward a body cavity at a time of operating the forward water-supplying button. Thereby, the body liquid which has adhered to the site to be examined within the body cavity or the like can be cleaned.

Observation conducted by the second imaging unit 30 for ordinary observation is continued until the distal end portion of the endoscope 2 inserted into the body of the patient is led to a targeted site to be observed. The control switch 112a is turned ON in a state that the distal end portion of the endoscope 2 has approached the targeted site to be observed so that switching to an observation mode with a high magnification using the first imaging unit 28 of an object-contacting type is performed.

When switching to the observation mode with a high magnification has been conducted, the distal end portion 15 of the insertion portion 11 is pressed on a surface of a body tissue H. At this time, the projecting step portion 25 of the distal end cover 24 is mainly pressed on the surface of the body tissue H, while a non-projecting portion except for the projecting step portion 25 is kept in a contact state with the surface of the body tissue H. Therefore, the first lens 41a and the illumination lens 91 of the first illumination window 29 at the distal end of the first imaging unit 28 disposed on the projecting step portion 25 are brought in contact with the surface of the body tissue H such as a cell tissue to be observed. Thereby, observation of the body tissue H such as a cell tissue in front of the first lens 41a of the first imaging unit 28 is performed in the observation mode with a high magnification using the first imaging unit 28 of an object-contacting type.

With the endoscope with the abovementioned configuration, the following effects can be obtained. That is, in the embodiment, since the air-supplying/water-supplying nozzle 34 that delivers fluid to the first lens 61a, which is the observation lens for the second imaging unit 30, is disposed at the low step portion 27 of the distal end portion 15 of the insertion portion 11, the height of the air-supplying/water-supplying nozzle 34 can be reduced. Thereby, when observation is conducted in a state that the flat face 25a of the projecting step portion 25 at the distal end portion 15 of the insertion portion 11 is in contact with a body tissue (a body to be examined), such a possibility can be reduced that the air-supplying/water-supplying nozzle 34 is caught by living tissue. Therefore, such a possibility can be reduced that the jetting port 34a of the distal end opening portion of the air-supplying/water-supplying nozzle 34 is caught by an object to be observed and such a possibility can be reduced that a surface of a body tissue positioned at a site to be observed is injured, so that observation or diagnosis work utilizing the first imaging unit 28 of a object-contacting type can be conducted easily.

Further, in the present embodiment, as shown in FIG. 12, since a distal end face of the jetting port 34a of the distal end opening portion of the air-supplying/water-supplying nozzle 34 and the first lens 61a, which is the observation lens for the second imaging unit 30, are disposed so as to be approximately flush with each other, such an effect that an excellent draining property is achieved can be obtained at a time of cleaning.

As shown in FIG. 12, since the jetting port 34a of the distal end opening portion of the air-supplying/water-supplying nozzle 34 is disposed behind the projecting face, which is the flat face 25a of the projecting step portion 25, when the flat face 25a of the projecting step portion 25 is caused to abut on a body to be examined, such a possibility can be reduced that the distal end portion of the air-supplying/water-supplying nozzle 34 is caught by the body to be examined, and a body tissue surface at the site to be observed can be reliably prevented from being injured.

In the present embodiment, since the area of the flat face 25a of the projecting step portion 25 contacting an object is set to be larger than that of the first embodiment, the first lens 41a for the first imaging unit 28 with a super-high magnification can be supported relatively stably when the projecting step portion 25 is pressed on a surface of a body tissue H, which is an object. Therefore, shaking of an observation image of the first imaging unit 28 or focus error can be reduced so that stable cell observation can be conducted.

Since the first lens 41a which is the observation lens for the first imaging unit 28, the first illumination window 29, and the distal end opening portion 33a of the procedure tool insertion channel 33 are disposed on the flat face 25a of the projecting step portion 25, a body tissue (a body to be examined) H can be pulled toward the flat face 25a of the projecting step portion 25 by conducting suction from the distal end opening portion 33a of the procedure tool insertion channel 33. Therefore, further stabilized observation with a super-high magnification can be made possible.

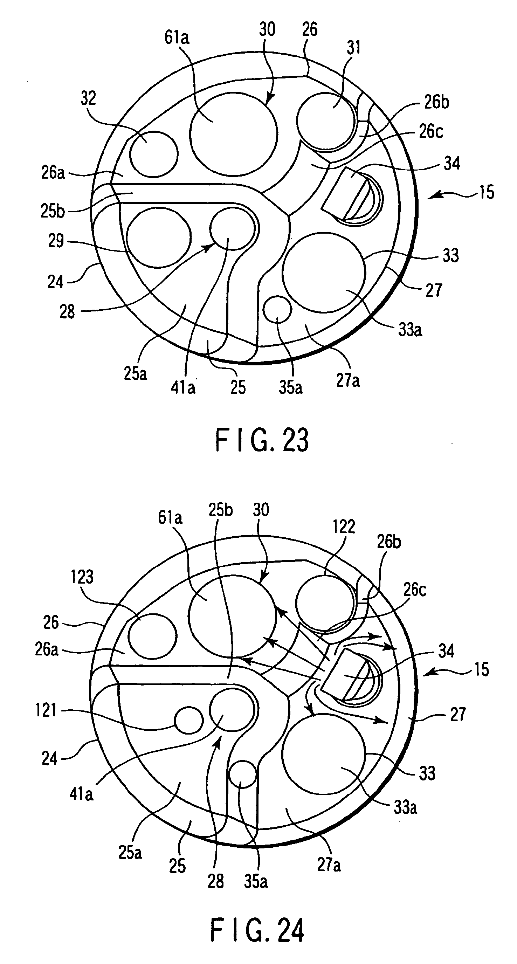

FIG. 23 shows a third embodiment of the present invention. The present embodiment has a configuration in which the endoscope 2 of the endoscope system 1 according to the first embodiment (see FIGS. 1 to 18) has been changed in the following manner.

That is, in the endoscope 2 according to the present embodiment, an opening portion 35a for forward water supply is disposed on a flat face 27a of a low step portion 27 on a front face of a distal end cover 24. Incidentally, the remaining portion of the endoscope 2 according to the third embodiment, except for the abovementioned portion, has the same configuration as that of the endoscope 2 according to the first embodiment, where the same portions of the endoscope 2 as those of the endoscope 2 according to the first embodiment are attached with the same reference numerals and explanation thereof is omitted.

With the abovementioned configuration, the following effects can be achieved. That is, in the present embodiment, since the air-supplying/water-supplying nozzle 34 that delivers fluid to the first lens 61a, which is the observation lens for the second imaging unit 30, is disposed at the low step portion 27 of the distal end portion 15 of the insertion portion 11, the height of the air-supplying/water-supplying nozzle 34 can be reduced. Thereby, when observation is conducted in a state that the flat face 25a of the projecting step portion 25 at the distal end portion 15 of the insertion portion 11 is in contact with a body tissue (a body to be examined), such a possibility can be reduced that the air-supplying/water-supplying nozzle 34 is caught by a living body. Therefore, such a possibility can be reduced that the jetting port 34a of the distal end opening portion of the air-supplying/water-supplying nozzle 34 is caught by an object, so that observation or diagnosis work utilizing the first imaging unit 28 of an object-contacting type can be easily conducted.

The air-supplying/water-supplying nozzle 34 and the opening portion 35a for forward water-supplying are provided on the same flat face as the distal end opening portion 33a of the procedure tool insertion channel 33 in parallel, water pooling around the air-supplying/water-supplying nozzle 34 or the opening portion 35a for forward water supply can be sucked via the distal end opening portion 33a of the procedure tool insertion channel 33 at a time of water supplying from the air-supplying/water-supplying nozzle 34 or the opening portion 35a for forward water supply. Therefore, any adverse influence on observation due to remaining water pooling around the air-supplying/water-supplying nozzle 34 or the opening portion 35a for forward water supply can be reduced.

FIG. 24 shows a fourth embodiment of the present invention. The present embodiment has a configuration in which the endoscope 2 of the endoscope system 1 according to the first embodiment (see FIGS. 1 to 18) has been changed in the following manner.

That is, in the endoscope 2 according to the present embodiment, a first lens 41a, which is an observation lens for a first imaging unit (a first imaging section) 28 with a high magnification, and a first illumination window 121 with the smallest illumination window area are disposed on a flat face 25a of a projecting step portion 25 on a front face of a distal end cover 24.

A first lens 61a, which is an observation lens for a second imaging unit (a second observation section) for ordinary observation, and two (first and second) illumination windows 122 and 123 are disposed on a flat face 26a of a middle step portion 26. Here, the second and third illumination windows 122 and 123 are disposed on both sides of a second imaging unit 30.

Further, regarding the second and third illumination windows 122 and 123 disposed on the flat face 26a of the middle step portion 26, setting is conducted such that the second illumination window 122 has the largest area, the third illumination window 123 has the second-largest area, and the first illumination window 121 on the flat face 25a of the projecting step portion 25 has the smallest area. Thereby, regarding light emission amounts from the three illumination windows, setting is performed such that the light emission amount from the second illumination window 122 is the largest amount, the light emission amount from the third illumination window 123 is the second-largest amount, and the light emission amount from the first illumination window 121 is the smallest amount. Incidentally, the remaining portion of the endoscope 2 according to the fourth embodiment except for the abovementioned portion has the same configuration as that of the endoscope 2 according to the first embodiment, where the same portions as those of the endoscope 2 according to the first embodiment are attached with the same reference numerals and explanation thereof is omitted.

With the endoscope with the abovementioned configuration, the following effects can be obtained. That is, in the present embodiment, since the air-supplying/water-supplying nozzle 34 that delivers fluid to the first lens 61a, which is the observation lens for the second imaging unit 30, is disposed at the low step portion 27 of the distal end portion 15 of the insertion portion 11, the height of the air-supplying/water-supplying nozzle 34 can be reduced. Thereby, when observation is conducted in a state that the flat face 25a of the projecting step portion 25 at the distal end portion 15 of the insertion portion 11 is in contact with a body tissue (a body to be examined), such a possibility can be reduced that the air-supplying/water-supplying nozzle 34 is caught by a living tissue. Therefore, such a possibility can be reduced that the jetting port 34a of the distal end opening portion of the air-supplying/water-supplying nozzle 34 is caught by an object to be observed and such a possibility can be reduced that a surface of a body tissue positioned at a site to be observed is injured, so that observation or diagnosis work utilizing the first imaging unit 28 of a object-contacting type can be conducted easily.

The first illumination window 121 with the smallest area of the plurality of illumination windows is disposed near the first lens 41a, which is the observation window for the first imaging unit 28 with a high magnification. Thereby, since the first illumination window 121 and the first lens 41a, which is the observation window for the first imaging unit 28, can be arranged so as to be close to each other, attenuation of illumination light for an observation image to be observed by the first imaging unit 28 can be suppressed. Therefore, such an effect can be obtained that color reproducibility of an observation image to be observed by the first imaging unit 28 is improved.

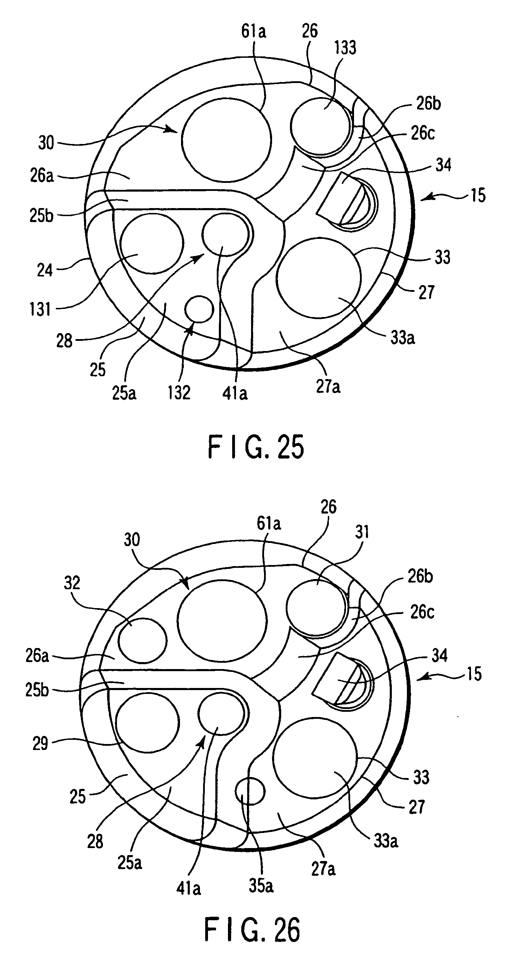

FIG. 25 shows a fifth embodiment of the present invention. The present embodiment has a configuration in which the endoscope 2 of the endoscope system 1 according to the first embodiment (see FIGS. 1 to 18) has been changed in the following manner.

That is, in the endoscope 2 according to the present embodiment, a first lens 41a, which is an observation lens for a first imaging unit (first observation section) 28 with a high magnification, and two (first and second) illumination windows 131 and 132 are disposed on a flat face 25a of a projecting step portion 25 on a front face of a distal end cover 24. Here, the first lens 41a is disposed at an approximately central portion of the distal end cover 24, and a first illumination window 131 with the largest illumination window area is disposed on a left side of the first lens 41a and a second illumination window 132 with the smallest illumination window area is disposed below the side of the first lens 41a in FIG. 25. As the second illumination window 132, a light source, for example, a small-sized light emitting diode (LED), which can be turned ON/OFF by a switch (not shown) is used.

A first lens 61a, which is an observation lens for a second imaging unit (second observation section) 30 for ordinary observation, and a third illumination window 133 are disposed on a flat face 26a of a middle step portion 26. Here, the first lens 61a is disposed on an upper position side of the first lens 41a in FIG. 25 and the third illumination window 133 is disposed on a right side of the second imaging unit 30 in FIG. 25.

Further, setting is performed such that the third illumination window 133 disposed on the flat face 26a of the middle step portion 26 has an area smaller than that of the first illumination window 131 and larger than that of the second illumination window 132. Incidentally, in the present embodiment, an opening portion 35a communicating with a pipe conduit 35 for forward water supply is provided on a non-projecting face of the projecting step portion 25 except for the flat face 25a. The remaining portion of the endoscope 2 except for the abovementioned portion has the same configuration as that of the endoscope 2 according to the first embodiment, where the same portions of the endoscope 2 according to the fifth embodiment as those of the endoscope 2 according to the first embodiment are attached with the same reference numerals and explanation thereof is omitted.

With the endoscope with the abovementioned configuration, the following effects can be obtained. That is, in the present embodiment, since the air-supplying/water-supplying nozzle 34 that delivers fluid to the first lens 61a, which is the observation lens for the second imaging unit 30, is disposed at the low step portion 27 of the distal end portion 15 of the insertion portion 11, the height of the air-supplying/water-supplying nozzle 34 can be reduced. Thereby, when observation is conducted in a state that the flat face 25a of the projecting step portion 25 at the distal end portion 15 of the insertion portion 11 is brought in contact with a body tissue (a body to be examined), such a possibility can be reduced that the air-supplying/water-supplying nozzle 34 is caught by a living tissue. Therefore, such a possibility can be reduced that the jetting port 34a of the distal end opening portion of the air-supplying/water-supplying nozzle 34 is caught by an object to be observed and such a possibility can be reduced that a surface of a body tissue positioned at a site to be observed is injured, so that observation or diagnosis work utilizing the first imaging unit 28 of an object-contacting type can be easily conducted.

Further, the two (first and second) illumination windows 131 and 132 are disposed on the flat face 25a of the projecting step portion 25 on a front face of the distal end cover 24 and the light source that can be turned ON/OFF is used as the second illumination window 132. Therefore, illumination lights from three illumination windows 131, 132, and 133 are emitted to an object during ordinary observation conducted using the second imaging unit 30, so that the object can be observed under bright illumination lights.

When the flat face 25a of the projecting step portion 25 on a front face of the distal end cover 24 is pressed on a surface of a body tissue H so that the first lens 41a at the distal end of the first imaging unit 28 is brought in contact with a surface of a body tissue H to be observed where the body tissue H such as a cell tissue is observed with a high magnification, the object can be illuminated with only illumination light from the first illumination window 131 by turning OFF one, for example, the second illumination window 132, of two (first and second) illumination windows 131 and 132 on the flat face 25a of the projecting step portion 25. Thereby, since the body tissue H on a body to be examined is not illuminated with light with a plurality of spectra, observation of a body tissue (a body to be examined) can be performed with excellent color reproducibility. Incidentally, such a configuration can be adopted that a plurality of illumination windows for LEDs are provided on the projecting step portion 25 and when a body tissue H is observed with a high magnification, only illumination of an LED light source for high magnification observation is utilized.

Further, when a body tissue H is observed with a high magnification, since illumination light at a time of contact is light which has transmitted through the body tissue H so that the illumination light is strongly influenced by scattering inside a live body, which is different from light utilized during ordinary observation, where when a distance between the observation window and the illumination window is large, an image to be obtained becomes dark reddish (wavelength is long). Therefore, the influence of scattering can be reduced by reducing a distance between the first lens 41a and the first illumination window 131 at the distal end of the first imaging unit 28, which is the observation window.

FIG. 26 shows a sixth embodiment of the present invention. The present embodiment has a configuration where the configuration of the endoscope 2 of the endoscope system 1 according to the first embodiment (see FIGS. 1 to 18) has been changed in the following manner.

That is, in the endoscope 2 according to the present embodiment, as shown in FIG. 26, a opening portion 35a for forward water supply is disposed between an inclined face 25b between a projecting step portion 25 and a low step portion 27 on a front face of a distal end cover 24 and a flat face 27a of the low step portion 27. Incidentally, the remaining portion of the endoscope according to the sixth embodiment has the same configuration as that of the endoscope 2 according to the first embodiment except for the abovementioned configuration. The same portions as those of the endoscope 2 according to the first embodiment are attached with the same reference numerals, and explanation thereof is omitted.

With the endoscope with the abovementioned configuration, the following effects can be obtained. That is, in the present embodiment, since the air-supplying/water-supplying nozzle 34 that delivers fluid to the first lens 61a that is the observation lens of the second imaging unit 30 is disposed at the low step portion 27 of the distal end portion 15 of the insertion portion 11, the height of the air-supplying/water-supplying nozzle 34 can be reduced. Thereby, when observation is conducted in a state that the flat portion 25a of the projecting step portion 25 at the distal end portion 15 of the insertion portion 11 is brought in contact with a body tissue (a body to be examined), such a possibility can be reduced that the air-supplying/water-supplying nozzle 34 is caught by a living body. Therefore, such a possibility can be reduced that the jetting port 34a of the distal end opening portion of the air-supplying/water-supplying nozzle 34 is caught by an object and such a possibility can be reduced that a surface of a body tissue positioned at a site to be observed is injured, so that observation or diagnosis work utilizing the first imaging unit 28 of a object-contacting type can easily be conducted.

Since the opening portion 35a communicating with a pipe conduit 35 for forward water supply is disposed between the inclined face 25b between the projecting step portion 25 and the low step portion 27 on the front face of the distal end cover 24 and the flat face 27a of the lower step portion 27, when a body tissue H is observed with a high magnification, a possibility can be reduced that, when the projecting step portion 25 on the front face of the distal end cover 24 is brought in contact with a body tissue H, the opening portion 35a for forward water supply comes in contact with the body tissue H. Therefore, such a possibility can be reduced that residue Q or the like clogs the opening portion 35a for forward water supply so that clogging of the opening portion 35a can be reduced.

Further, water pooling around the air-supplying/water-supplying nozzle 34 or the opening portion 35a for forward water supply can be sucked via the distal end opening portion 33a of the procedure tool insertion channel 33 at a time of water-supplying from the air-supplying/water-supplying nozzle 34 or the opening portion 35a for forward water supply. Therefore, any adverse influence on observation due to remaining water pooling around the air-supplying/water-supplying nozzle 34 or the opening portion 35a for forward water supply can be reduced.

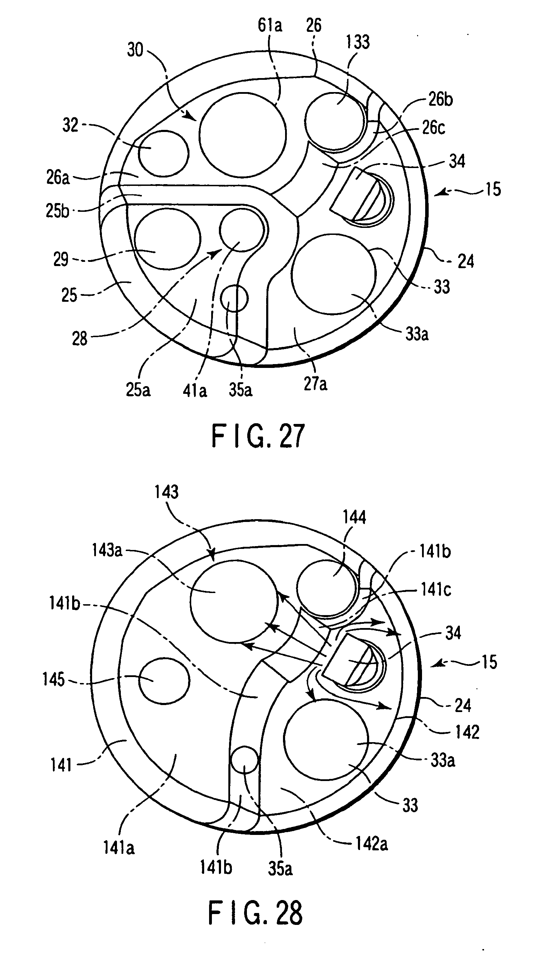

FIG. 27 shows a seventh embodiment of the present invention. The present embodiment has a configuration in which the endoscope 2 of the endoscope system 1 according to the first embodiment (see FIGS. 1 to 18) has been changed in the following manner.

That is, in the endoscope 2 according to the present embodiment, as shown in FIG. 27, an opening portion 35a for forward water supply is disposed between an inclined face 25b between a projecting step portion 25 and a middle step portion 26 on a front face of a distal end cover 24 and a flat face 25a of the projecting step portion 25. Incidentally, the endoscope according to the seventh embodiment has the same configuration as that of the endoscope 2 according to the first embodiment except for the abovementioned configuration. The same portions as those of the endoscope 2 according to the first embodiment are attached with the same reference numerals, and explanation thereof is omitted.

With the endoscope with the abovementioned configuration, the following effects can be obtained. That is, in the present embodiment, since the air-supplying/water-supplying nozzle 34 that delivers fluid to the first lens 61a, which is the observation lens for the second imaging unit 30, is disposed at the low step portion 27 of the distal end portion 15 of the insertion portion 11, the height of the air-supplying/water-supplying nozzle 34 can be reduced. Thereby, when observation is conducted in a state that the flat face 25a of the projecting step portion 25 at the distal end portion 15 of the insertion portion 11 is brought in contact with a body tissue (a body to be examined), such a possibility can be reduced that the air-supplying/water-supplying nozzle 34 is caught by a living body. Therefore, such a possibility can be reduced that the jetting port 34a of the distal end opening portion of the air-supplying/water-supplying nozzle 34 is caught by an object and such a possibility can be reduced that a surface of a body tissue positioned at a site to be observed is injured, so that observation or diagnosis work utilizing the first imaging unit 28 of an object-contacting type can be easily conducted.

Since the opening portion 35a for forward water supply is disposed between the inclined face 25b between the projecting step portion 25 and the middle step portion 26 on the front face of the distal end cover 24 and the flat face 25a of the projecting step portion 25, a portion of the opening portion 35a for forward water supply is provided to extend on the inclined face 25b. Therefore, since an area of the opening portion 35a for forward water supply can be enlarged as compared with a case that the opening portion 35a for forward water supply is formed on a whole area of the flat face 25a of the projecting step portion 25 on the front face of the distal end cover 24, such a possibility can be reduced that residue Q or the like clogs the opening portion 35a for forward water supply so that clogging of the opening portion 35a can be reduced.

Further, a rod-shaped tool 114 can be easily inserted into the opening portion 35a on the inclined face 25b. Therefore, even if the opening portion 35a at the distal end portion of a pipe conduit 35 for forward water supply is clogged with residue Q or the like, clogging of the opening portion 35a of the pipe conduit 35 for forward water supply due to residue Q or the like can be easily remedied by inserting the rod-shaped tool 114 into the opening portion 35a. As a result, a distal end portion of an endoscope 2 where clogging of the opening portion 35a of the pipe conduit 35 for forward water supply can be prevented and endoscope observation is facilitated can be provided.

FIG. 28 shows an example useful for understanding the present invention. The present embodiment has a configuration in which the endoscope 2 of the endoscope system 1 according to the first embodiment (see FIGS. 1 to 18) has been changed in the following manner. Incidentally, same portions as those of the endoscope 2 according to the first embodiment are attached with the same reference numerals and explanation thereof is omitted.

That is, as shown in FIG. 28, in the endoscope 2 according to the present embodiment, a projecting step portion 141 projecting forward and a low step portion 142 lower than the projecting step portion 141 by one step are provided on a front face of a distal end cover 24. Here, an end face of the projecting step portion (projecting portion) 141 is formed by a flat face 141a perpendicular to an axial direction of an insertion portion 11. A projecting face is formed by the flat face 141a of the projecting step portion 141.

In the present embodiment, the flat face 141a of the projecting step portion 141 is formed to have an area of about 1/2 of a whole circular front face of the distal end cover 24. That is, the flat face 141a is formed on a left side portion relative to a centerline connecting upper and lower portions of the front face so as to have an area of one half of the whole front circular face of the distal end cover 24.

A first lens 143a, which is an observation lens for an imaging unit 143 provided with a zoom optical system that can conduct zoom action from an ordinary observation position to an enlargement observation position with a high magnification, and two (first and second) illumination windows 144 and 145 are disposed on the flat face 141a of the projecting step portion 141. Here, the imaging unit 143 is disposed on an approximately central and upper end position of the distal end portion 15 in FIG. 28. The first illumination window 144 is disposed on a left side position of the imaging unit 143 and the second illumination window 145 is disposed on a right side position of the imaging unit 143. Further, in the present embodiment, an area of the first illumination window 144 is set to be larger than that of the second illumination window 145.

The low step portion 142 has a flat face 142a approximately parallel to the flat face 141a of the projecting step portion 141. A distal end opening portion 33a of a procedure tool insertion channel (also called "forceps channel") 33 disposed inside the insertion portion 11 and an air-supplying/water-supplying nozzle 34 are disposed on the flat face 142a of the low step portion 142.

Further, an inclined face 141b with an inclination angle of, for example, about 45° and a fluid guide face 141c with an inclination angle smaller than that of the inclined face 141b are formed on a wall portion between the low step portion 142 and the projecting step portion 141. The fluid guide face 141c is disposed between an air-supplying/water-supplying nozzle 34 of the low step portion 142 and the first lens 143a for the imaging unit 143 of the projecting step portion 141. The fluid guide face 141c is formed by an inclined face with a gentle inclination angle of, for example, about 18°.

An opening portion 35a for forward water supply is disposed on the inclined face 141b between the low step portion 142 and the projecting step portion 141. The opening portion 35a communicates with a pipe conduit for forward water supply (forward water supplying channel) 35 inserted into the insertion portion 11.

An operation of the endoscope system 1 with the abovementioned configuration will be explained. The imaging unit 143 can be selectively switched between an ordinary observation state and an observation state with a high magnification of an object-contacting type at a time of using the endoscope 2 according to the present embodiment. The imaging unit 143 is switched to the ordinary observation state during an insertion work for inserting the endoscope 2 into a patient's body. In this case, ordinary observation for observing an object that is spaced from the first lens 143a for the imaging unit 143 in a wide range is conducted using the imaging unit 143.

When matter such as body liquid or extraneous matter adheres to a surface of the first lens 143 for the imaging unit 143 during the ordinary observation, the air-supplying/water-supplying button 109 is operated. Air supply and water supply are conducted through an air supplying pipe conduit 106a and a water supplying pipe conduit 106b by operation of the air-supplying/water-supplying button 109. A gas such as air or a liquid such as sterile water is jetted from a jetting port 34a of an air-supplying/water-supplying nozzle 34 on the low step portion 27 of the distal end cover 24 in a jetting direction. At this time, a fluid such as sterile water or air jetted from the jetting port 34a of the air-supplying/water-supplying nozzle 34 is guided toward the first lens 143a side for the imaging unit 143 along a fluid guide face 141c of a projecting step portion 141 and matter such as body liquid or extraneous matter that has adhered to a surface of the first lens 143a for the imaging unit 143 is removed and cleaned so that imaging and the observation field of view can be maintained in a clean state.

Further, when body fluid adheres to a site to be examined in a body cavity so that the site is soiled, the forward water-supplying button is operated. A liquid such as sterile water is sprayed from the opening portion 35a of the distal end cover 24 of the insertion portion 11 in an insertion direction toward a body cavity at a time of operating the forward water-supplying button. Thereby, body liquid which has adhered to the site to be examined within the body cavity or the like can be cleaned.

Observation conducted by the imaging unit 143 for ordinary observation is continued until the distal end portion of the endoscope 2 is led to a targeted site to be observed. The control switch 112a is turned ON in a state that the distal end portion 15 of the endoscope 2 has approached the targeted site to be observed so that switching to an observation mode with a high magnification using the imaging unit 143 of an object-contacting type is performed.

When the zoom optical system of the imaging unit 143 is switched to an observation mode with a high magnification in this manner, the distal end portion 15 of the insertion portion 11 is pressed on a surface of a body tissue H. At this time, the projecting step portion 141 of the distal end cover 24 is mainly pressed on the surface of the body tissue H, while a non-projecting face, except for the projecting step portion 141, is kept in a contacted state with the surface of the body tissue H. Therefore, the first lens 143a and the respective illumination lens of the first illumination window 144 and the second illumination window 145 at the distal end of the imaging unit 143 disposed on the projecting step portion 141 are brought in contact with the surface of the body tissue H such as a cell tissue to be observed. Thereby, observation of the body tissue H such as a cell tissue in front of the first lens 143a for the imaging unit 143 is performed in the observation mode with a high magnification using the imaging unit 143 as an optical system of an object-contacting type.

With the endoscope with the abovementioned configuration, the following effects can be obtained. That is, in the present embodiment, since the air-supplying/water-supplying nozzle 34 that delivers fluid to the first lens 143a, which is the observation lens of the imaging unit 143, is disposed at the low step portion 142, which is lower than the projecting step portion 141 on a front face of the distal end portion 15 of the insertion portion 11 by one step, the height of the air-supplying/water-supplying nozzle 34 can be reduced. Thereby, when observation is conducted in a state that the flat portion 141a of the projecting step portion 141 at the distal end portion 15 of the insertion portion 11 is brought in contact with a body tissue (a body to be examined), such a possibility can be reduced that the air-supplying/water-supplying nozzle 34 is caught by living tissue. Therefore, such a possibility can be reduced that the jetting port 34a of the distal end opening portion of the air-supplying/water-supplying nozzle 34 is caught by an object and such a possibility can be reduced that a surface of a body tissue positioned at a site to be observed is injured, so that observation or diagnosis work utilizing the imaging unit 143 of an object-contacting type can be easily conducted.

In the present embodiment, since the projecting step portion 141 and the low step portion 142 are provided on the front face of the distal end cover 24, and the first lens 143a, which is the observation lens for the imaging unit 143 provided with the zoom optical system that allows zoom action from the ordinary observation position to the enlargement observation position with a high magnification and the first and second illumination windows 144 and 145, are disposed on the flat face 141a of the projecting step portion 141, the overall space required for the imaging unit can be reduced as compared with a case of an imaging unit for ordinary observation and an imaging unit with a high magnification for enlargement observation are individually provided. Therefore, size reduction and diameter reduction of the distal end portion 15 of the endoscope 2 can be achieved.

Further, the present invention is not limited to the abovementioned embodiments and can naturally be implemented in variously-modified forms without departing from the gist of the present invention.

Industrial Applicability

The present invention is effective in a technical field in which an endoscope is inserted into, for example, a body cavity and is provided with an observation optical system for ordinary observation and an observation optical system of an object-contacting type where an object is observed while a distal end portion of an object optical system is being brought in contact with the object, as well as the technical field of endoscope manufacturing.

**Claims**

1. A distal end portion of an endoscope comprising:

   an insertion portion (11) to be inserted into a body to be examined;
   a projecting face (25a) which is provided at a distal end portion of the insertion portion (11) in a projecting manner and on which a first observation portion (28) for observing the body to be examined is disposed;
   a non-projecting face (26a, 27a) which is provided at the insertion portion (11) and has a flat face (27a) approximately parallel to the projecting face (25a);
   a second observation portion (30) which is disposed on the non-projecting face (26a, 27a); and
   a step- formed between the projecting face (25a) and the non-projecting face (26a, 27a), step having height which prevents the projecting face (25a) from entering a field of view of the second observation portion (30);
   **characterized in that** a nozzle portion (34) is provided on the non-projecting face (26a, 27a) for delivering fluid to the second observation portion (30).

2. The distal end portion of an endoscope according to claim 1, wherein the nozzle portion (34) comprises at least one of nozzles for air supply and for water supply.

3. The distal end portion of an endoscope according to claim 1, wherein a distal end of the nozzle portion (34) is disposed on a proximal end side rather than the projecting face.

4. The distal end portion of an endoscope according to claim 1, wherein the first observation portion (28) has a magnification which allows observation of a structure of a body tissue, and is configured to have a magnification of about 200 times to about 1,000 times.

5. The distal end portion of an endoscope according to claim 4, wherein the first observation portion (28) has an observation depth of about 0 to 100 μm, and is an observation portion of a contact type for performing observation in a contact state with a body to be examined.

6. The distal end portion of an endoscope according to claim 1, wherein the step is set to about 0.7 mm.

7. The distal end portion of an endoscope according to claim 1, wherein the distal end portion of the insertion portion has, on a corner portion end edge portion on a distal end portion side face on an opposite side spaced from an arrangement position of the first observation portion, a chamfer angle enlargement portion with a chamfer angle larger than that of a corner portion end edge portion on the other side face.

8. The distal end portion of an endoscope according to claim 7, wherein the chamfer angle enlargement portion is set such that a chamfer angle R is in a range of about 1 mm to about 1.3 mm, while the corner portion end edge portion on the side face other than the chamfer angle enlargement portion is set such that the chamfer angle R is in a range of about 0.7 mm to about 1 mm.

9. The distal end portion of an endoscope according to claim 1, wherein
the projecting face is disposed with an illumination portion (29) for emitting illumination light to the body to be examined, and
front end portions of the first observation portion (28) and the illumination portion (29) project forward beyond a flat face position of the projecting face.

10. The distal end portion of an endoscope according to claim 9, wherein
the front end portion of the illumination portion (29) projects forward beyond a front end portion position of the first observation portion (28).

11. A distal end portion of an endoscope according to claim 1, wherein
the non-projecting face includes a first distal end face (26a) which is provided at a. distal end portion of the insertion portion (11) and on which the second observation portion (30) for observing the body to be examined is disposed; and
a third distal end face (27a) which is provided on a proximal end side to the first distal end face (26a); and wherein
the projecting face includes a second distal end face (25a) which is provided on a distal end side to the first distal end face (26a) in a projecting manner and on which the first observation portion (28) for observing the body to be examined is disposed; and wherein
the nozzle portion (34) provided on the third distal end face (27a) for delivering a fluid to the second observation portion (30).

12. The distal end portion of an endoscope according to claim 11, wherein the nozzle portion comprises at least one of nozzles for air supply and for water supply.

13. The distal end portion of an endoscope according to claim 11, wherein a distal end of the nozzle portion is disposed on a proximal end side to the second distal end face.

14. The distal end portion of an endoscope according to claim 11, wherein the nozzle portion has an opening portion, and a distal end side of the opening portion and the second observation portion are disposed to be approximately flush with each other.

15. The distal end portion of an endoscope according to claim 11, wherein at least a portion of the first distal end face has a flat face approximately parallel to the second distal end face, and
a step between the first distal end face and the second distal end face is formed to have a height by which the second distal end face can be prevented from entering in a field of view of the second observation portion.

16. The distal end portion of an endoscope according to claim 15, wherein the step is set to about 0.7 mm.

17. The distal end portion of an endoscope according to claim 11, wherein the first observation portion is an observation portion with a magnification higher than that of the second observation portion.

18. The distal end portion of an endoscope according to claim 11, wherein the first observation portion has an observation depth of about 0 to 100 μm, and is an observation portion for performing observation in a contact state with a body to be examined.

19. The distal end portion of an endoscope according to claim 11, wherein the distal end portion of the insertion portion has, on a corner portion end edge portion on a distal end portion side face on an opposite side spaced from an arrangement position of the observation portion, a chamfer angle enlargement portion with a chamfer angle larger than that of a corner portion end edge portion on the other side face.

20. The distal end portion of an endoscope according to claim 19, wherein the chamfer angle enlargement portion is set such that a chamfer angle R is in a range of about 1 mm to about 1.3 mm, while the corner portion end edge portion on the side face other than the chamfer angle enlargement portion is set such that the chamfer angle R is in a range of about 0.7 mm to about 1 mm.

**Patentansprüche**

1.  Distaler Endbereich eines Endoskops mit:

    einem Einführbereich (11), der in einen zu untersuchenden Körper einzuführen ist;
    einer Überstandfläche (25a), die an einem distalen Endbereich des Einführbereichs (11) in hervorstehender Art und Weise vorgesehen ist und an der ein erster Beobachtungsbereich (28) zum Beobachten des zu untersuchenden Körpers angeordnet ist;
    einer nicht überstehenden Fläche (26a, 27a), die am Einführbereich (11) vorgesehen ist und etwa parallel zur Überstandfläche (25a) eine ebene Fläche (27a) aufweist;
    einem zweiten Beobachtungsbereich (30), der auf der nicht überstehenden Fläche (26a, 27a) angeordnet ist; und
    einer Stufe, die zwischen der Überstandfläche (25a) und der nicht überstehenden Fläche (26a, 27a) ausgebildet ist und eine Höhe aufweist, welche verhindert, dass die Überstandfläche (25a) in ein Sichtfeld des zweiten Beobachtungsbereichs (30) hineinragt;
    **dadurch gekennzeichnet,**
    **dass** ein Düsenbereich (34) an der nicht überstehenden Fläche (26a, 27a) vorgesehen ist, um dem zweiten Beobachtungsbereich (30) ein Fluid zu liefern.

2.  Distaler Endbereich eines Endoskops nach Anspruch 1, wobei der Düsenbereich (34) eine Düse zum Zuführen von Luft und/oder eine Düse zum Zuführen von Wasser umfasst.

3.  Distaler Endbereich eines Endoskops nach Anspruch 1, wobei ein distales Ende des Düsenbereichs (34) und nicht die Überstandsfläche auf einer proximalen Endseite angeordnet ist.

4.  Distaler Endbereich eines Endoskops nach Anspruch 1, wobei der erste Beobachtungsbereich (28) eine Vergrößerung aufweist, welche eine Beobachtung einer Struktur eines Körpergewebes gestattet, und so ausgebildet ist, dass er eine etwa 200-fache bis etwa 1000-fache Vergrößerung aufweist.

5.  Distaler Endbereich eines Endoskops nach Anspruch 4, wobei der erste Beobachtungsbereich (28) eine Beobachtungstiefe von etwa 0 bis 100 $\mu$m aufweist und ein Beobachtungsbereich des Kontakttyps ist, um eine Beobachtung im Zustand des Kontakts mit einem zu untersuchenden Körper auszuführen.

6.  Distaler Endbereich eines Endoskops nach Anspruch 1, wobei die Stufe auf etwa 0,7 mm festgelegt ist.

7.  Distaler Endbereich eines Endoskops nach Anspruch 1, wobei der distale Endbereich des Einführbereichs an einem Eckbereich-Endkantenbereich an einer distalen Endbereich-Seitenfläche auf einer gegenüberliegenden Seite, die zu einer Anordnungsposition des ersten Beobachtungsbereichs beabstandet ist, einen Abkantungswinkel-Aufweitungsbereich mit einem Abkantungswinkel aufweist, der größer als der eines Eckbereich-Endkantenbereichs an der anderen Seitenfläche ist.

8.  Distaler Endbereich eines Endoskops nach Anspruch 7, wobei der Abkantungswinkel-Aufweitungsbereich so festgelegt ist, dass ein Abkantungswinkel R innerhalb eines Bereichs von etwa 1 mm bis etwa 1,3 mm liegt, während der Eckbereich-Endkantenbereich an der Seitenfläche im Gegensatz zum Abkantungswinkel-Aufweitungsbereich so festgelegt ist, dass der Abkantungswinkel R in einem Bereich von etwa 0,7 mm bis etwa 1 mm liegt.

9.  Distaler Endbereich eines Endoskops nach Anspruch 1, wobei die Überstandfläche mit einem Beleuchtungsbereich (29) versehen ist, um Beleuchtungslicht zu dem zu untersuchenden Körper auszusenden, und vordere Endbereiche des ersten Beobachtungsbereichs (28) und des Beleuchtungsbereichs (29) über die Position einer ebenen Fläche der Überstandfläche hinaus nach vorne vorstehen.

10. Distaler Endbereich eines Endoskops nach Anspruch 9, wobei der vordere Endbereich des Beleuchtungsbereichs (29) über eine vordere Endbereichsposition des ersten Beobachtungsbereichs (28) hinaus nach vorne vorsteht.

11. Distaler Endbereich eines Endoskops nach Anspruch 1, wobei
    die nicht überstehende Fläche eine erste distale Endfläche (26a) aufweist, die an einem distalen Endbereich des Einführbereichs (11) vorgesehen ist und an welcher der zweite Beobachtungsbereich (30) zum Beobachten des zu untersuchenden Körpers angeordnet ist; und
    eine dritte distale Endfläche (27a) an einer proximalen Endseite zur ersten distalen Endfläche (26a) vorgesehen ist; und wobei
    die Überstandfläche eine zweite distale Endfläche (25a) aufweist, die an einer distalen Endseite zur ersten distalen Endfläche (26a) in einer vorstehenden Art und Weise vorgesehen ist und an welcher der erste Beobachtungsbereich (28) zum Beobachten des zu untersuchenden Körpers angeordnet ist; und wobei

der Düsenbereich (34) an der dritten distalen Endfläche (27a) zum Liefern eines Fluides an den zweiten Beobachtungsbereich (30) vorgesehen ist.

12. Distaler Endbereich eines Endoskops nach Anspruch 11, wobei der Düsenbereich eine Düse zum Zuführen von Luft und/oder eine Düse zum Zuführen von Wasser umfasst.

13. Distaler Endbereich eines Endoskops nach Anspruch 11, wobei ein distales Ende des Düsenbereichs an einer proximalen Endseite zu der zweiten distalen Endfläche angeordnet ist.

14. Distaler Endbereich eines Endoskops nach Anspruch 11, wobei der Düsenbereich einen Öffnungsbereich aufweist und eine distale Endseite des Öffnungsbereichs und der zweite Beobachtungsbereich so angeordnet sind, dass sie zueinander in etwa bündig abschließen.

15. Distaler Endbereich eines Endoskops nach Anspruch 11, wobei wenigstens ein Bereich der ersten distalen Endfläche eine ebene Fläche aufweist, die in etwa parallel zur zweiten distalen Endfläche ist, und

eine Stufe zwischen der ersten distalen Endfläche und der zweiten distalen Endfläche so ausgebildet ist, dass sie eine Höhe aufweist, durch welche die zweite distale Endfläche daran gehindert werden kann, in ein Sichtfeld des zweiten Beobachtungsbereichs hineinzuragen.

16. Distaler Endbereich eines Endoskops nach Anspruch 15, wobei die Stufe auf etwa 0,7 mm festgelegt ist.

17. Distaler Endbereich eines Endoskops nach Anspruch 11, wobei der erste Beobachtungsbereich ein Beobachtungsbereich mit einer Vergrößerung ist, die über der des zweiten Beobachtungsbereichs liegt.

18. Distaler Endbereich eines Endoskops nach Anspruch 11, wobei der erste Beobachtungsbereich eine Beobachtungstiefe von etwa 0 bis 100 pm aufweist und ein Beobachtungsbereich ist, der eine Beobachtung im Zustand des Kontakts mit einem zu untersuchenden Körper ausführen kann.

19. Distaler Endbereich eines Endoskops nach Anspruch 11, wobei der distale Endbereich des Einführbereichs an einem Eckbereich-Endkantenbereich an einer distalen Endbereich-Seitenfläche auf einer gegenüberliegenden Seite, die zu einer Anordnungsposition des Beobachtungsbereichs beabstandet ist, einen Abkantungswinkel-Aufweitungsbereich mit einem Abkantungswinkel aufweist, der größer als der eines Eckbereich-Endkantenbereichs auf der anderen Seitenfläche ist.

20. Distaler Endbereich eines Endoskops nach Anspruch 19, wobei der Abkantungswinkel-Aufweitungsbereich so festgelegt ist, dass ein Abkantungswinkel R innerhalb eines Bereichs von etwa 1 mm bis etwa 1,3 mm liegt, während der Eckbereich-Endkantenbereich an der Seitenfläche im Gegensatz zum Abkantungswinkel-Aufweitungsbereich so festgelegt ist, dass der Abkantungswinkel R in einem Bereich von etwa 0,7 mm bis etwa 1 mm liegt.

**Revendications**

1. Partie d'extrémité distale d'un endoscope comprenant :

une partie d'insertion (11) devant être insérée dans un corps devant être examiné ;
une face se projetant (25a) qui est prévue au niveau d'une partie d'extrémité distale de la partie d'insertion (11) d'une manière faisant saillie et sur laquelle une première partie d'observation (28) destinée à observer le corps devant être examiné est disposée ;
une face ne se projetant pas (26a, 27a) qui est prévue au niveau de la partie d'insertion (11) et possède une face plate (27a) approximativement parallèle à la face se projetant (25a) ;
une deuxième partie d'observation (30) qui est disposée sur la face ne se projetant pas (26a, 27a) ; et
une marche formée entre la face se projetant (25a) et la face ne se projetant pas (26a, 27a) et présentant une hauteur qui empêche la face se projetant (25a) d'entrer dans un champ de vision de la deuxième partie d'observation (30) ;
**caractérisée en ce que**
une partie de buse (34) est prévue sur la face ne se projetant pas (26a, 27a) destinée à délivrer un fluide vers la deuxième partie d'observation (30).

2. Partie d'extrémité distale d'un endoscope selon la revendication 1, dans laquelle la partie de buse (34) comprend au moins l'une parmi des buses d'alimentation en air et d'alimentation en eau.

3. Partie d'extrémité distale d'un endoscope selon la revendication 1, dans laquelle une extrémité distale de la partie de buse (34) est disposée sur un côté d'extrémité proximale plutôt que sur la face se projetant.

4. Partie d'extrémité distale d'un endoscope selon la revendication 1, dans laquelle la première partie d'observation (28) présente un grossissement qui permet une observation d'une structure d'un tissu de corps, et est configurée pour présenter un grossissement d'environ 200 fois à environ 1 000 fois.

5. Partie d'extrémité distale d'un endoscope selon la revendication 4, dans laquelle la première partie d'observation (28) présente une profondeur d'observation d'environ 0 à 100 $\mu$m, et est une partie d'observation d'un type avec contact destinée à réaliser une observation dans un état de contact avec un corps devant être examiné.

6. Partie d'extrémité distale d'un endoscope selon la revendication 1, dans laquelle la marche est établie à environ 0,7 mm.

7. Partie d'extrémité distale d'un endoscope selon la revendication 1, dans laquelle la partie d'extrémité distale de la partie d'insertion comporte, sur une partie de bord d'extrémité de partie de coin sur une face latérale de la partie d'extrémité distale sur un côté opposé espacé d'une position de disposition de la première partie d'observation, une partie d'agrandissement d'angle de chanfrein avec un angle de chanfrein supérieur à celui d'une partie de bord d'extrémité de partie de coin sur l'autre face latérale.

8. Partie d'extrémité distale d'un endoscope selon la revendication 7, dans laquelle la partie d'agrandissement d'angle de chanfrein est établie d'une manière telle qu'un angle de chanfrein R se situe dans une plage allant d'environ 1 mm à environ 1,3 mm, alors que la partie de bord d'extrémité de partie de coin sur la face latérale autre que la partie d'agrandissement d'angle de chanfrein est établie d'une manière telle que l'angle de chanfrein R se situe dans une plage allant d'environ 0,7 mm à environ 1 mm.

9. Partie d'extrémité distale d'un endoscope selon la revendication 1, dans laquelle la face se projetant est disposée avec une partie d'éclairage (29) destinée à émettre une lumière d'éclairage sur le corps devant être examiné, et les parties d'extrémité avant de la première partie d'observation (28) et de la partie d'éclairage (29) se projettent vers l'avant au-delà d'une position de face plate de la face se projetant.

10. Partie d'extrémité distale d'un endoscope selon la revendication 9, dans laquelle la partie d'extrémité avant de la partie d'éclairage (29) se projette vers l'avant au-delà d'une position de partie d'extrémité avant de la première partie d'observation (28).

11. Partie d'extrémité distale d'un endoscope selon la revendication 1, dans laquelle

la face ne se projetant pas comprend une première face d'extrémité distale (26a) qui est prévue au niveau d'une partie d'extrémité distale de la partie d'insertion (11) et sur laquelle la deuxième partie d'observation (30) destinée à observer le corps devant être examiné est disposée ; et une troisième face d'extrémité distale (27a) qui est prévue sur un côté d'extrémité proximal par rapport à la première face d'extrémité distale (26a) ; et dans laquelle la face se projetant comprend une deuxième face d'extrémité distale (25a) qui est prévue sur un côté d'extrémité distal par rapport à la première face d'extrémité distale (26a) d'une manière faisant saillie et sur laquelle la première partie d'observation (28) destinée à observer le corps devant être examiné est disposée ; et dans laquelle la partie de buse (34) est prévue sur la troisième face d'extrémité distale (27a) destinée à délivrer un fluide vers la deuxième partie d'observation (30).

12. Partie d'extrémité distale d'un endoscope selon la revendication 11, dans laquelle la partie de buse comprend au moins l'une parmi des buses d'alimentation en air et d'alimentation en eau.

13. Partie d'extrémité distale d'un endoscope selon la revendication 11, dans laquelle une extrémité distale de la partie de buse est disposée sur un côté d'extrémité proximal par rapport à la deuxième face d'extrémité distale.

14. Partie d'extrémité distale d'un endoscope selon la revendication 11, dans laquelle la partie de buse comporte une partie d'ouverture, et un côté d'extrémité distale de la partie d'ouverture et la deuxième partie d'observation sont disposés pour être approximativement de niveau l'un avec l'autre.

15. Partie d'extrémité distale d'un endoscope selon la revendication 11, dans laquelle au moins une partie de la première face d'extrémité distale comporte une face plate approximativement parallèle à la deuxième face d'extrémité distale, et une marche entre la première face d'extrémité distale et la deuxième face d'extrémité distale est formée pour présenter une hauteur de laquelle la deuxième face d'extrémité distale peut être empêchée d'entrer dans un champ de vision de la deuxième partie d'observation.

16. Partie d'extrémité distale d'un endoscope selon la revendication 15, dans laquelle la marche est établie à environ 0,7 mm.

17. Partie d'extrémité distale d'un endoscope selon la revendication 11, dans laquelle la première partie d'observation est une partie d'observation avec un grossissement plus élevé que celui de la deuxième partie d'observation.

**18.** Partie d'extrémité distale d'un endoscope selon la revendication 11, dans laquelle la première partie d'observation présente une profondeur d'observation allant d'environ 0 à 100 μm, et est une partie d'observation destinée à réaliser une observation dans un état de contact avec un corps devant être examiné.

**19.** Partie d'extrémité distale d'un endoscope selon la revendication 11, dans laquelle la partie d'extrémité distale de la partie d'insertion comporte, sur une partie de bord d'extrémité de partie de coin sur une face latérale de la partie d'extrémité distale sur un côté opposé espacé d'une position de disposition de la partie d'observation, une partie d'agrandissement d'angle de chanfrein avec un angle de chanfrein supérieur à celui d'une partie de bord d'extrémité de partie de coin sur l'autre face latérale.

**20.** Partie d'extrémité distale d'un endoscope selon la revendication 19, dans laquelle la partie d'agrandissement d'angle de chanfrein est établie d'une manière telle qu'un angle de chanfrein R se situe dans une plage allant d'environ 1 mm à environ 1,3 mm, alors que la partie de bord d'extrémité de partie de coin sur la face latérale autre que la partie d'agrandissement d'angle de chanfrein est établie d'une manière telle que l'angle de chanfrein R se situe dans une plage allant d'environ 0,7 mm à environ 1 mm.

FIG.1

FIG. 2

FIG. 3

FIG. 4A

FIG. 4B

F I G. 5A

F I G. 5B

FIG. 6

F I G. 7

EP 1 880 656 B1

FIG. 8A

EP 1 880 656 B1

F I G. 8B

F I G. 8C

F I G. 9A

FIG. 9B

EP 1 880 656 B1

FIG.10

FIG. 11A

EP 1 880 656 B1

F I G. 11B

F I G. 12

F I G. 13

F I G. 14

FIG.15

15

O4

15a

24

91

29

O3

L

H

41a

202

201

26

30

25

28

FIG. 16A

25a

x

y

θ

30

t

26a

61a

25

FIG. 16B

FIG. 17

FIG. 18

F I G. 19

F I G. 20

F I G. 21

F I G. 22

FIG. 23

FIG. 24

F I G. 25

F I G. 26

FIG. 27

FIG. 28

**EP 1 880 656 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5782751 A
- US 5860913 A
- US 5730701 A
- US 20040158129 A